# EUROPEAN PATENT APPLICATION

(11) **EP 4 624 488 A1**
(43) Date of publication of application: **01.10.2025**
(21) Application number: 23894911.9
(22) Date of filing: 14.11.2023
(51) Int. Cl.: C07K 14/50, A61K 38/18, A61P 1/16

(54) **USE OF FGF5 TRANSCRIPT VARIANT-2 GENE AND PROTEIN THEREOF FOR PREVENTING, INHIBITING, AND TREATING LIVER FIBROSIS**

(30) Priority: 25.11.2022 KR 20220159875; 13.11.2023 KR 20230156822
(71) Applicant: Gil Medical Center, Incheon 21565 (KR); Gachon University of Industry-Academic Cooperation Foundation, Seongnam-si, Gyeonggi-do 13120 (KR)
(72) Inventor: LEE, Dae Ho, Incheon 22009 (KR); OH, Byung Chul, Incheon 21982 (KR)
(74) Representative: Regimbeau
(86) International application number: PCT/KR2023/018303
(87) International publication number: WO 2024/111982

(57) **Abstract**

Provided is the use of an FGF5-v2 (FGF5 transcript variant 2) gene and a protein thereof in preventing or treating liver fibrosis. The FGF5-v2 fragment gene (transcript and protein expressed therefrom) of the present disclosure is a mutated gene in which an amino acid sequence of fibroblast growth factor 5 (FGF5) is modified, and has the effect of inhibiting the progression and proliferation of liver fibrosis. Therefore, the FGF5-v2 is expected to prevent and treat liver fibrosis, thereby preventing the progression thereof to cirrhosis and liver cancer, when administered to a patient with fatty liver through a vector (e.g., AVV) or the like, and thus may be effectively used as a composition for treating liver fibrosis.

## Description

### [Technical Field]

The present disclosure relates to the use of a fibroblast growth factor 5 (FGF5) transcript variant and a protein expressed therefrom, etc. in preventing, inhibiting, or treating liver fibrosis.

The present disclosure is based on, and claims priority from, Korean Patent Application Nos. 10-2022-0159875 and 10-2023-0156822, filed on November 25, 2022 and November 13, 2023, respectively, the disclosures disclosed in the specifications and drawings of the corresponding applications are hereby incorporated by reference herein in their entirety.

### [Background Art]

Liver fibrosis results from the excessive accumulation of extracellular matrix, including collagen, that occurs in most types of chronic liver diseases. Representative cells involved in liver fibrosis include hepatic stellate cells, Kupffer cells, endothelial cells, etc. Hepatic stellate cells are the main source of extracellular matrix and are involved in the increased production of various extracellular matrices including collagen. Kupffer cells reside in the sinusoidal space of the liver, and substances produced by activated Kupffer cells affect the surrounding hepatocytes, endothelial cells, and hepatic stellate cells, thereby promoting liver fibrosis. Endothelial cells play a critical role in regulating blood flow within the liver, and also participate in the production of growth factors and extracellular matrix that are involved in the proliferation of hepatic stellate cells due to inflammation or liver fibrosis. When liver fibrosis occurs, activated hepatic stellate cells increase exponentially, and these cells secrete profibrogenic cytokines, which in turn produce extracellular matrix-related molecules such as α-SMA, collagen, and tissue inhibitor of metalloproteinases (TIMPs).

In particular, non-alcoholic fatty liver disease (NAFLD) among liver diseases is a disease that progresses to non-alcoholic steatohepatitis (NASH) and cirrhosis, and is highly related to metabolic diseases such as obesity, diabetes, and hyperlipidemia, rather than to drinking. Non-alcoholic steatohepatitis is recognized as a serious disease worldwide because it develops into cirrhosis and liver cancer which are caused by inflammation and liver fibrosis resulting from hepatocellular degeneration/necrosis due to the fat accumulation in hepatocytes. NASH is caused by two distinct events, evolving from steatosis to fibrosis, and this is called a "two-hit process" (Exp Anim. 2014; 63(1): 85-92.).

Meanwhile, fibroblast growth factor (FGF) is a type of growth factor that forms a family of 23 members, and each type is known to have various functions and activities in the body, and their specific roles have not been completely identified. In this situation, it was previously known that fibroblast growth factor 5 (FGF5) participates in the progression of liver fibrosis in non-alcoholic steatohepatitis (NASH) (Exp Anim. 2014; 63(1): 85-92.). However, the present inventors confirmed that a newly identified FGF5 variant is able to effectively suppress fibrosis in the liver, and based on this idea, they identified the potential of the FGF5 variant as a therapeutic agent for liver diseases based on its superior anti-fibrotic effect.

### [Disclosure]

### [Technical Problem]

Accordingly, the present inventors have studied a therapeutic strategy capable of controlling liver fibrosis, and they have newly found that, unlike the existing knowledge that fibroblast growth factor 5 (FGF5) participates in the progression of liver fibrosis in non-alcoholic steatohepatitis (NASH), FGF-v2 (FGF5 transcript variant 2) according to the present disclosure, which is an FGF5 transcript variant and a short variant of FGF5, remarkably inhibits liver fibrosis, thereby completing the present invention.

Accordingly, an object of the present disclosure is to provide an FGF5-v2 transcript variant and a protein thereof (also referred to as "FGF5-v2 protein"), consisting of an amino acid sequence of SEQ ID NO: 1 in the sequence listing.

Another object of the present disclosure is to provide a nucleic acid molecule (i.e., FGF5-v2 gene) encoding the FGF5-v2 protein.

Still another object of the present disclosure is to provide a carrier comprising the FGF5-v2 gene of the present disclosure, a transcript thereof, or a protein thereof (or loading the gene, transcript, or protein); or a composition comprising the carrier.

Still another object of the present disclosure is to provide a pharmaceutical composition for preventing or treating liver fibrosis (liver fibrotic disease), the pharmaceutical composition comprising, as an active ingredient, the FGF5-v2 protein consisting of the amino acid sequence of SEQ ID NO: 1 in the sequence listing of the present disclosure; an expression vector comprising the nucleic acid molecule encoding the FGF5-v2 protein; or an isolated cell, into which the expression vector is introduced.

However, the technical problems to be achieved by the present disclosure are not limited to the problems mentioned above, and other problems not mentioned may be clearly understood by those skilled in the art, to which the present invention belongs, from the description below.

### [Technical Solution]

To achieve the above objects, the present disclosure provides an FGF5-v2 protein consisting of an amino acid sequence of SEQ ID NO: 1 in the sequence listing.

Further, the present disclosure provides a nucleic acid molecule encoding the FGF5-v2 protein.

In one embodiment of the present disclosure, the FGF5-v2 gene may comprise a nucleotide sequence of SEQ ID NO: 2 in the sequence listing, but is not limited thereto.

Further, the present disclosure provides an expression vector comprising the nucleic acid molecule.

Further, the present disclosure provides a cell, into which the expression vector is introduced. Preferably, the cell is an isolated cell.

Further, the present disclosure provides a pharmaceutical composition for preventing or treating liver fibrosis, the pharmaceutical composition comprising, as an active ingredient, the FGF5-v2 protein consisting of the amino acid sequence of SEQ ID NO: 1 in the sequence listing; the nucleic acid molecule encoding the FGF5-v2 protein; the expression vector comprising the nucleic acid molecule; or the isolated cell, into which the expression vector is introduced.

In one embodiment of the present disclosure, the FGF5-v2 protein, the nucleic acid molecule, the expression vector, or the cell may be loaded into a carrier, but is not limited thereto.

In another embodiment of the present disclosure, the composition comprises the FGF5-v2 protein, the nucleic acid molecule, or the expression vector as an active ingredient, and the carrier may be one or more selected from the group consisting of a viral particle, a vesicle, a nanoparticle, a microparticle, a liposome, a transposon, a micelle, an antibody, and an exosome, but is not limited thereto.

In still another embodiment of the present disclosure, the liver fibrosis may be liver fibrosis in one or more selected from the group consisting of alcoholic fatty liver disease, non-alcoholic fatty liver disease, hepatitis C, hepatitis B, autoimmune hepatitis, hepatic encephalopathy, primary biliary cirrhosis, liver cancer, and hepatocellular carcinoma, but is not limited thereto.

In still another embodiment of the present disclosure, the non-alcoholic fatty liver disease may be any one or more selected from the group consisting of non-alcoholic steatohepatitis (NASH), non-alcoholic fatty liver (NAFL), hepatic steatosis, acute fatty liver of pregnancy (AFLP), NAFLD-related liver failure, NAFLD-related liver fibrosis, NAFLD-related hepatocirrhosis, and NAFLD-related liver cancer, but is not limited thereto.

In still another embodiment of the present disclosure, the composition may be administered in combination with an anti-inflammatory agent, but is not limited thereto.

In still another embodiment of the present disclosure, the anti-inflammatory agent may be a steroidal anti-inflammatory agent or a non-steroidal anti-inflammatory agent, but is not limited thereto.

In addition, the present disclosure provides a method of preventing or treating liver fibrosis, the method comprising the step of administering the FGF5-v2 protein consisting of the amino acid sequence of SEQ ID NO: 1 in the sequence listing; the nucleic acid molecule encoding the FGF5-v2 protein; the expression vector comprising the nucleic acid molecule; or the isolated cell, into which the expression vector is introduced, to an individual in need thereof. However, the FGF5-v2 gene (nucleic acid molecule), the transcript, and/or the protein of the present disclosure may be administered to an individual using various carriers known in the art, in addition to a vector. As the carrier, for example, expression vectors, viruses, vesicles, nanoparticles, microparticles, liposomes, transposons, micelles, exosomes, antibodies, etc. may be used.

In addition, the present disclosure provides use of a composition in preventing or treating liver fibrosis, the composition comprising, as an active ingredient, the FGF5-v2 protein consisting of the amino acid sequence of SEQ ID NO: 1; the nucleic acid molecule encoding the FGF5-v2 protein; the expression vector comprising the nucleic acid molecule; or the isolated cell, into which the expression vector is introduced.

In addition, the present disclosure provides use of a composition in preparing a prophylactic or therapeutic agent for liver fibrosis, the composition comprising, as an active ingredient, the FGF5-v2 protein consisting of the amino acid sequence of SEQ ID NO: 1; the nucleic acid molecule encoding the FGF5-v2 protein; the expression vector comprising the nucleic acid molecule; or the isolated cell, into which the expression vector is introduced.

In addition, the present disclosure provides use of, in preventing or treating liver fibrosis, the FGF5-v2 protein consisting of the amino acid sequence of SEQ ID NO: 1 in the sequence listing; the nucleic acid molecule encoding the FGF5-v2 protein; the expression vector comprising the nucleic acid molecule; or the isolated cell, into which the expression vector is introduced.

In addition, the present disclosure provides use of, in preparing a prophylactic or therapeutic agent for liver fibrosis, the FGF5-v2 protein consisting of the amino acid sequence of SEQ ID NO: 1 in the sequence listing; the nucleic acid molecule encoding the FGF5-v2 protein; the expression vector comprising the nucleic acid molecule; or the isolated cell, into which the expression vector is introduced.

### [Advantageous Effects]

FGF5-v2, which is a transcript variant of fibroblast growth factor 5 (FGF5), and a protein thereof according to the present disclosure was developed by adjusting the amino acid sequence of full-length FGF5. It was confirmed that the transcript variant and the protein thereof have effects of inhibiting the progression of liver fibrosis and improving liver function abnormalities accompanying liver fibrosis, etc. and various increased inflammation- and fibrosis-related cytokines, etc. Therefore, it is expected to achieve the effects of preventing, improving, and treating various diseases comprising cirrhosis or liver cancer, etc. by suppressing liver fibrosis by administering, to a patient, a gene encoding FGF5-v2, the transcript thereof, the protein thereof, and/or various carriers (e.g., AAV) loading the same.

### [Description of Drawings]

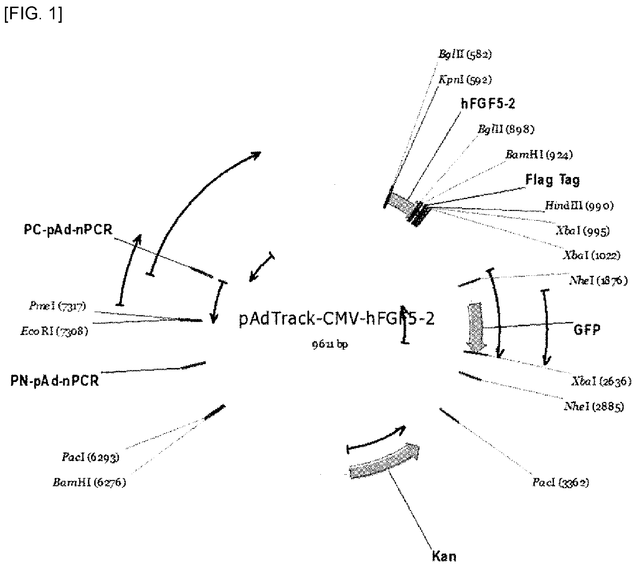

FIG. 1 shows a recombinant expression vector for intracellular delivery and expression (transcript or protein expression) of a gene encoding an FGF5-v2 variant (referred to as "FGF5-v2" or "FGF5-S") of the present disclosure;

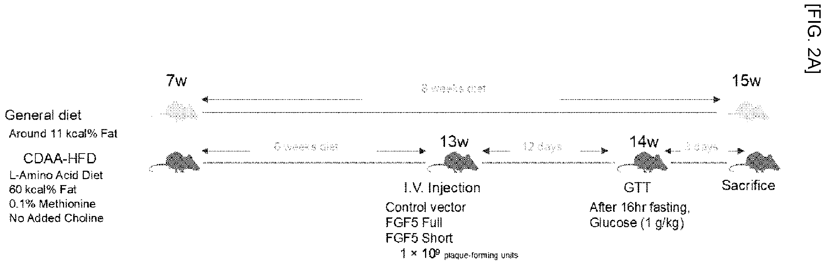

FIG. 2A is a schematic illustration showing a series of experimental schedules, in which *in vivo* models of liver fibrosis (CDAA-HFD induced *in vivo* NAFLD mouse models) were prepared, and the inhibitory effect of the FGF5-v2 variant (referred to as "FGF5-v2" or "FGF5-S") of the present disclosure on liver fibrosis was examined;

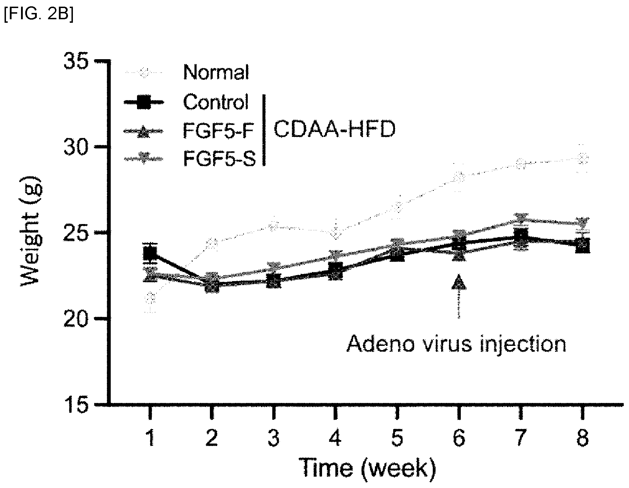

FIG. 2B shows results of examining changes in body weight over time after injecting a carrier comprising the gene of full-length FGF5 ("FGF5-F") or FGF5-v2 variant (referred to as "FGF5-v2" or "FGF5-S") into NAFLD mouse models (Normal, normal mouse; Control, untreated control group; the same applies hereinafter);

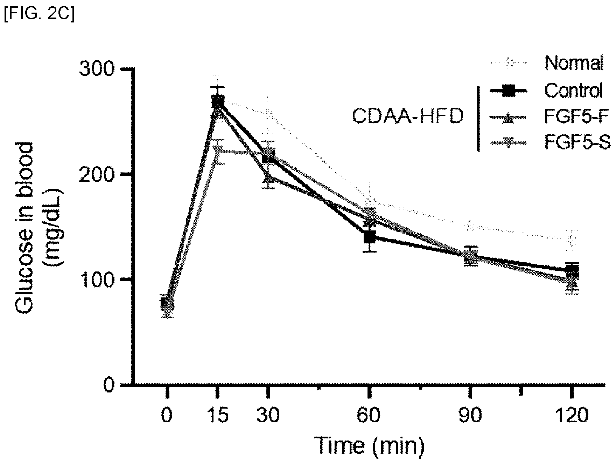

FIG. 2C shows changes in glucose tolerance in a normal mouse, a comparative group, an FGF5-F administered group, and an FGF5-v2 variant (referred to as "FGF5-v2" or "FGF5-S") administered group upon glucose injection after 12 hr fasting;

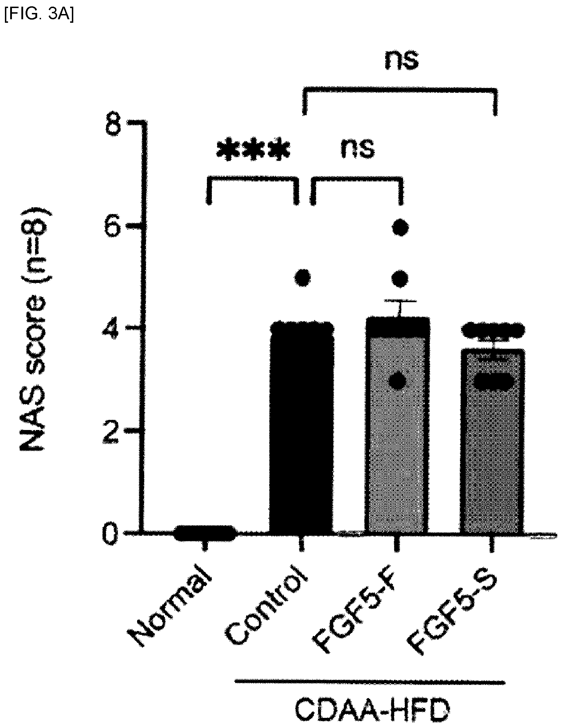

FIG. 3A shows results of comparing NAFLD activity scores (NAS) after injecting the gene of FGF5-F or FGF5-v2 variant (referred to as "FGF5-v2" or "FGF5-S") into NAFLD mouse models;

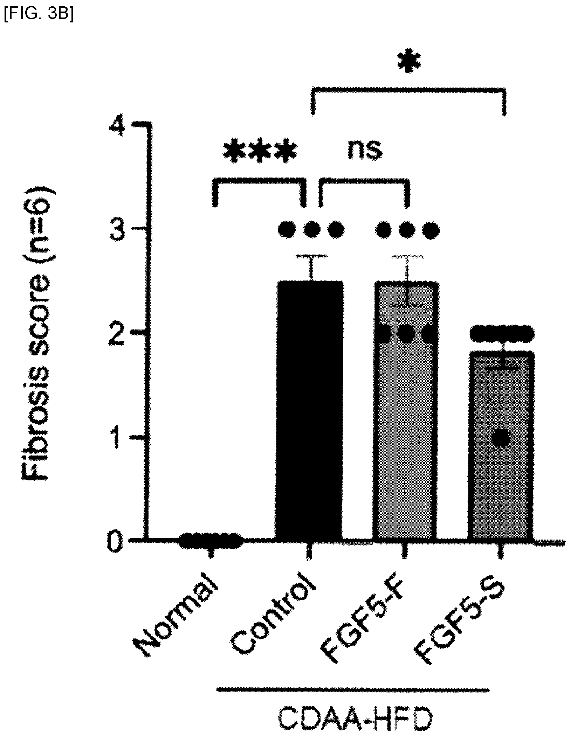

FIG. 3B shows results of comparing fibrosis scores after injecting the gene of FGF5-F or FGF5-v2 variant (referred to as "FGF5-v2" or "FGF5-S") into NAFLD mouse models, confirming the inhibitory effects on liver fibrosis by the specific injection of FGF5-v2 variant (referred to as "FGF5-v2" or "FGF5-S");

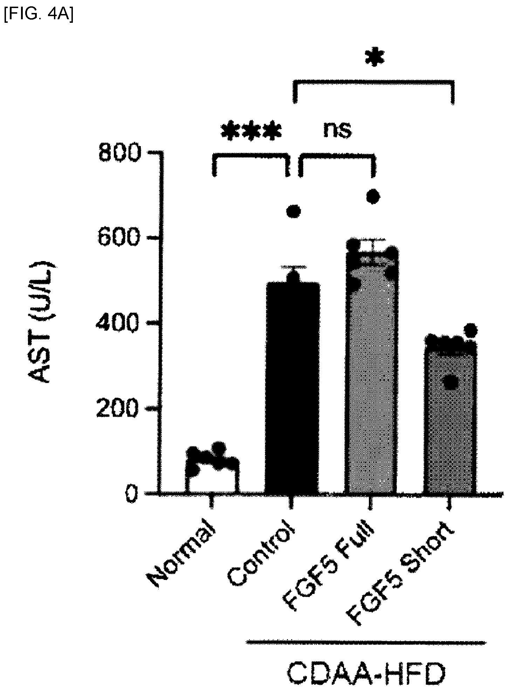

FIG. 4A shows results of comparing AST, which is an indicator of liver function, in the sera of NAFLD mouse models after injecting the gene of FGF5-F or FGF5-v2 variant (referred to as "FGF5-v2" or "FGF5-S") into NAFLD mouse models, confirming the inhibitory effects on AST (i.e., effects of improving liver damage and liver function) by the specific injection of FGF5-v2 variant (referred to as "FGF5-v2" or "FGF5-S");

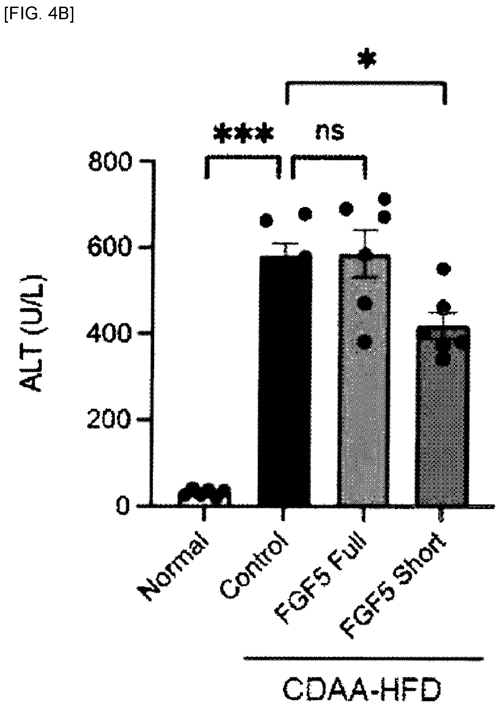

FIG. 4B shows the results of comparing ALT, which is a liver function test, in the sera of NAFLD mouse models after injecting the gene of FGF5-F or FGF5-v2 variant (referred to as "FGF5-v2" or "FGF5-S") into NAFLD mouse models, confirming the inhibitory effects on ALT (i.e., effects of improving liver damage and liver function) by the specific injection of FGF5-v2 variant (referred to as "FGF5-v2" or "FGF5-S");

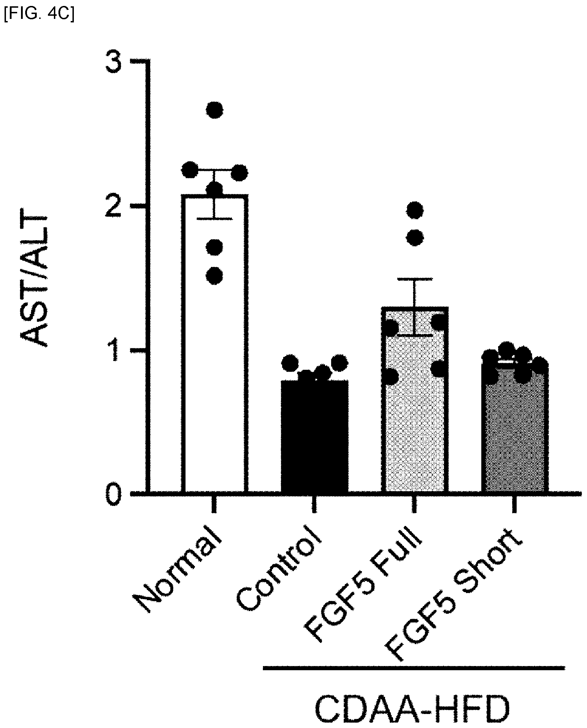

FIG. 4C shows the results of examining AST/ALT ratios in the sera of NAFLD mouse models after injecting the gene of FGF5-F or FGF5-v2 variant (referred to as "FGF5-v2" or "FGF5-S") into NAFLD mouse models;

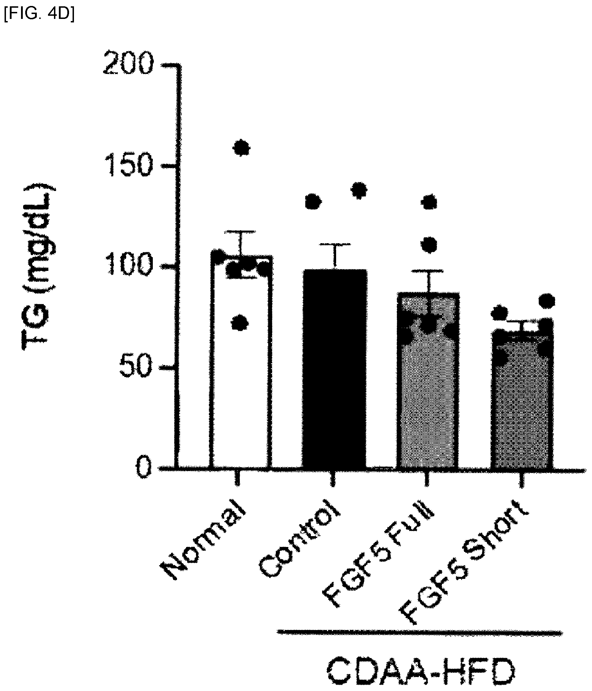

FIG. 4D shows the results of comparing levels of triglyceride (TG), among lipids, in the sera of NAFLD mouse models after injecting the gene of FGF5-F or FGF5-v2 variant (referred to as "FGF5-v2" or "FGF5-S") into NAFLD mouse models;

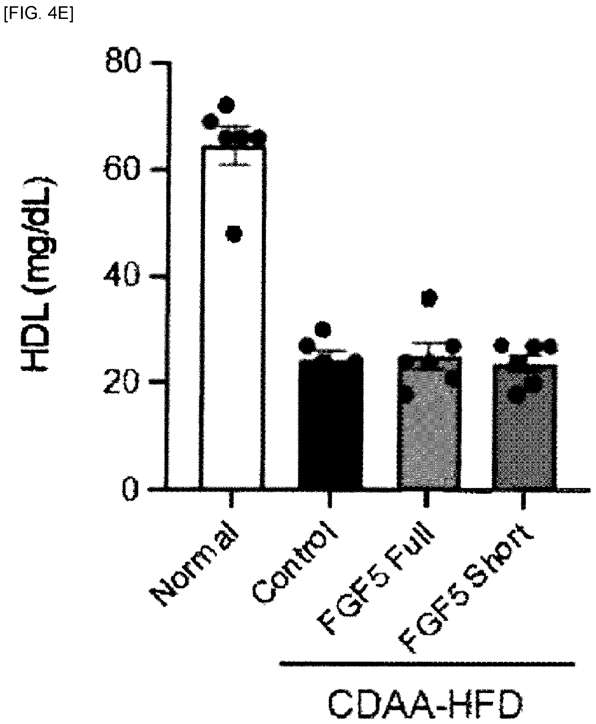

FIG. 4E shows a graph of comparing levels of high-density lipoprotein (HDL), among lipids, in the sera of NAFLD mouse models after injecting the gene of FGF5-F or FGF5-v2 variant (referred to as "FGF5-v2" or "FGF5-S") into NAFLD mouse models;

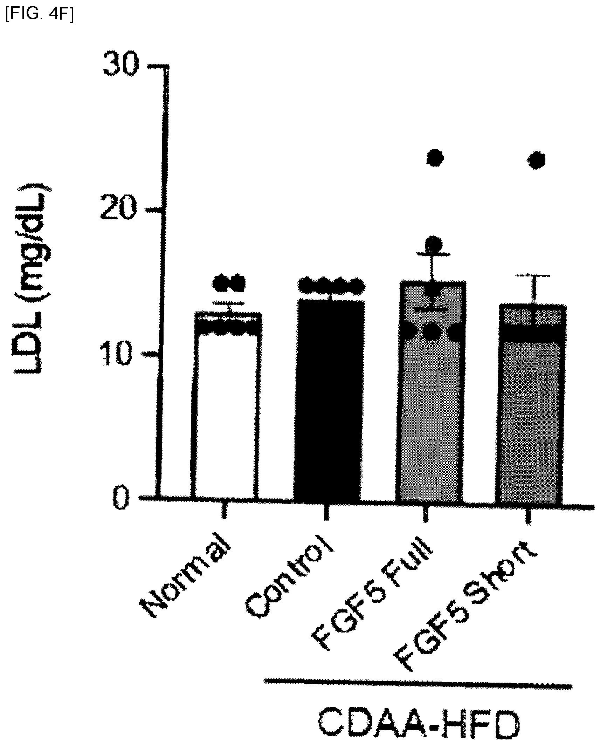

FIG. 4F shows a graph of comparing levels of low-density lipoprotein (LDL), among lipids, in the sera of NAFLD mouse models after injecting the gene of FGF5-F or FGF5-v2 variant (referred to as "FGF5-v2" or "FGF5-S") into NAFLD mouse models;

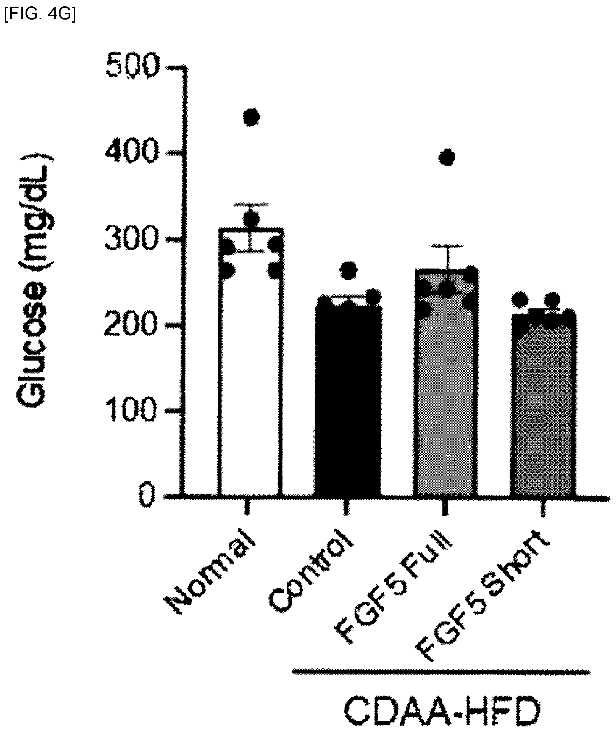

FIG. 4G shows a graph of comparing levels of glucose in the sera of NAFLD mouse models after injecting the gene of FGF5-F or FGF5-v2 variant (referred to as "FGF5-v2" or "FGF5-S") into NAFLD mouse models;

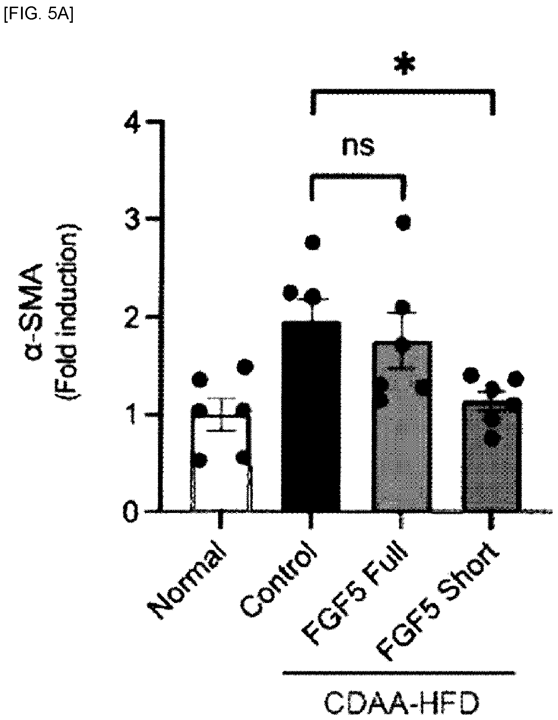

FIG. 5A shows a graph of comparing levels of α-SMA, among fibrosis-related biomarkers, in the liver tissue mRNA of NAFLD mouse models after injecting the gene of FGF5-F or FGF5-v2 variant (referred to as "FGF5-v2" or "FGF5-S") into NAFLD mouse models, confirming the inhibitory effects on α-SMA by the specific injection of FGF5-v2 variant (referred to as "FGF5-v2" or "FGF5-S") at the mRNA level;

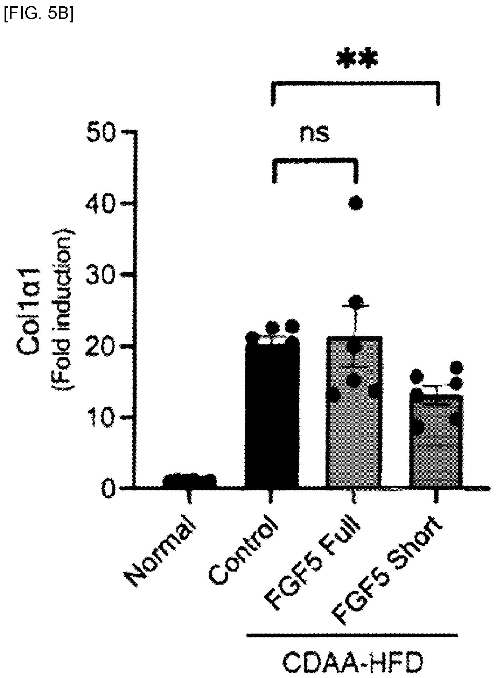

FIG. 5B shows a graph of comparing levels of Col1α1, among fibrosis-related biomarkers, in the liver tissue mRNA of NAFLD mouse models after injecting the gene of FGF5-F or FGF5-v2 variant (referred to as "FGF5-v2" or "FGF5-S") into NAFLD mouse models, confirming the inhibitory effects on Col1 by the specific injection of FGF5-v2 variant (referred to as "FGF5-v2" or "FGF5-S");

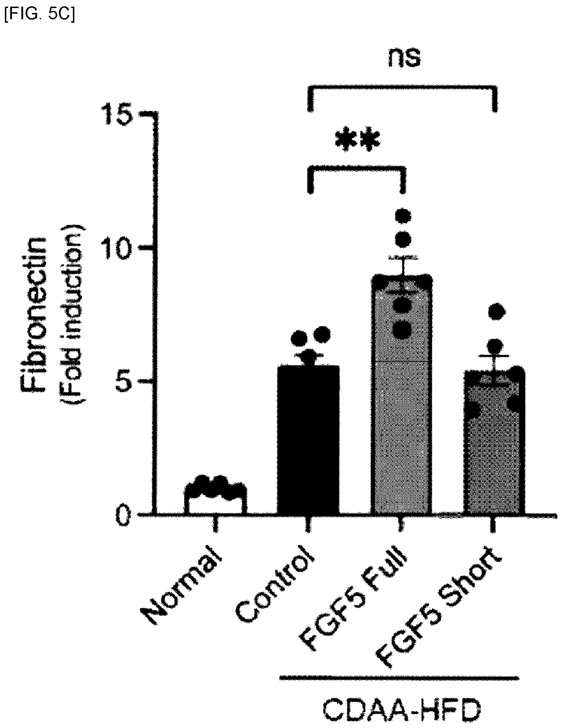

FIG. 5C shows a graph of comparing levels of fibronectin, among fibrosis-related biomarkers, in the liver tissue mRNA of NAFLD mouse models after injecting the gene of FGF5-F or FGF5-v2 variant (referred to as "FGF5-v2" or "FGF5-S") into NAFLD mouse models;

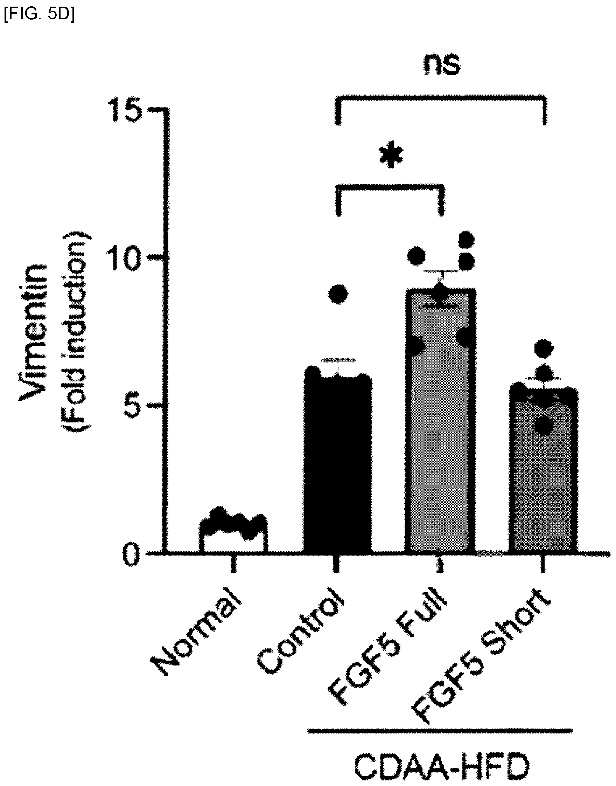

FIG. 5D shows a graph of comparing levels of vimentin, among fibrosis-related biomarkers, in the liver tissue mRNA of NAFLD mouse models after injecting the gene of FGF5-F or FGF5-v2 variant (referred to as "FGF5-v2" or "FGF5-S") into NAFLD mouse models;

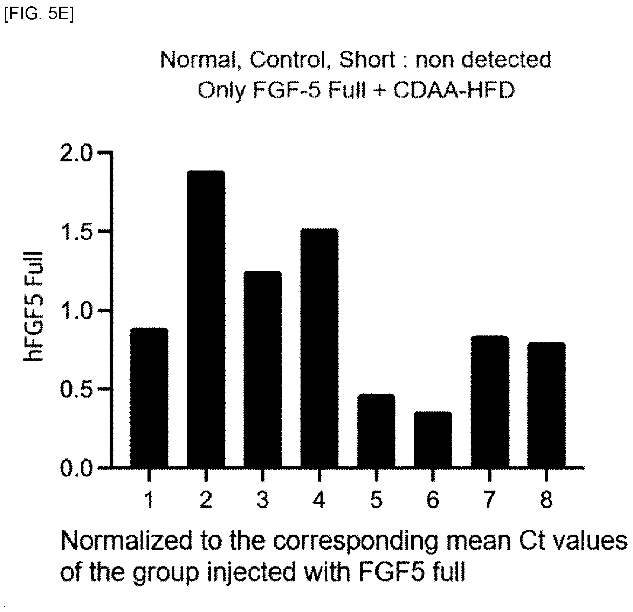

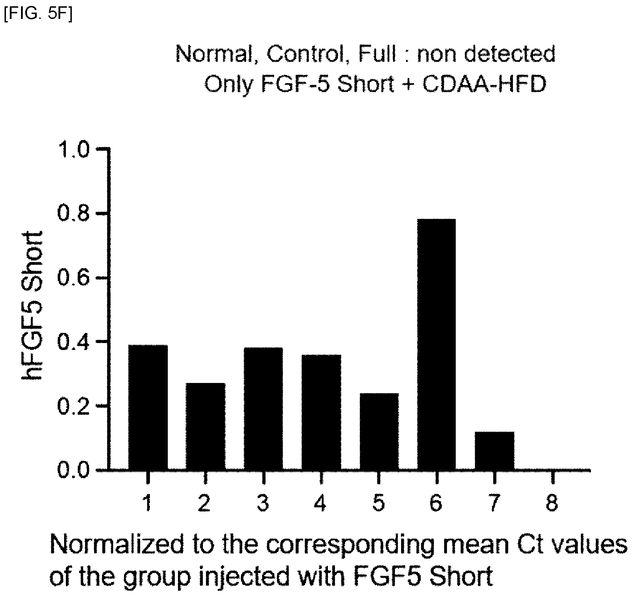

FIGS. 5E and 5F show the results of measuring expression levels of FGF5-F and FGF5-v2 variant (referred to as "FGF5-v2" or "FGF5-S") transcript in NAFLD mouse models (n=8 per group) into which the gene of FGF5-F (FIG. 5E) or FGF5-v2 variant (referred to as "FGF5-v2" or "FGF5-S") (FIG. 5F) was injected;

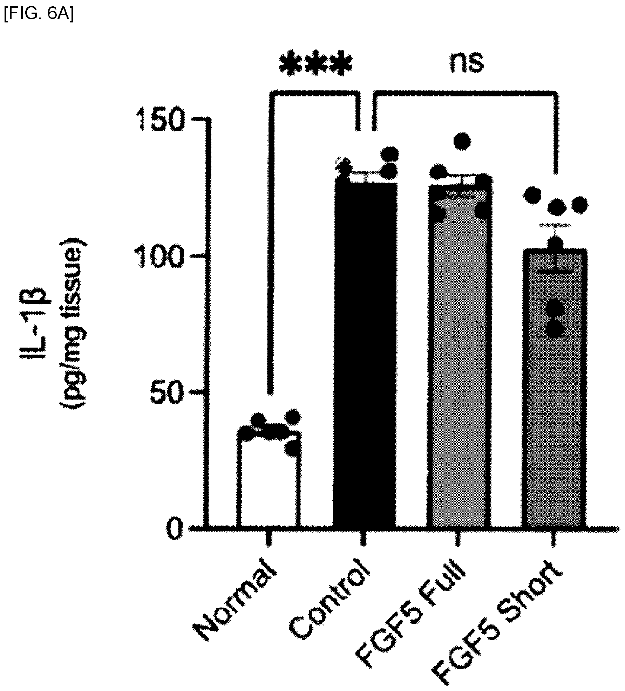

FIG. 6A shows the results of quantitative analysis of the expression of IL-1β protein, which is an inflammatory marker, through ELISA experiments using the liver tissues of NAFLD mouse models after injecting the gene of FGF5-F or FGF5-v2 variant (referred to as "FGF5-v2" or "FGF5-S") into NAFLD mouse models;

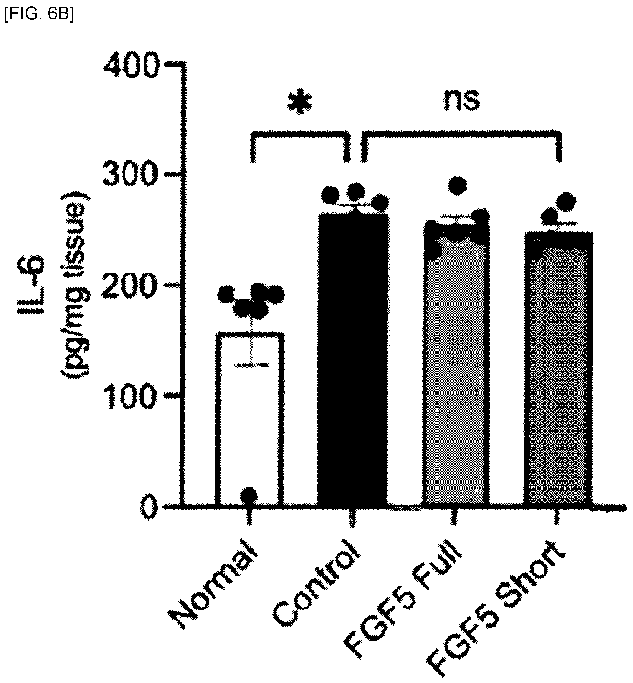

FIG. 6B shows results of quantitative analysis of the expression of IL-6 protein, which is an inflammatory marker, through ELISA experiments using the liver tissues of NAFLD mouse models after injecting the gene of FGF5-F or FGF5-v2 variant (referred to as "FGF5-v2" or "FGF5-S") into NAFLD mouse models;

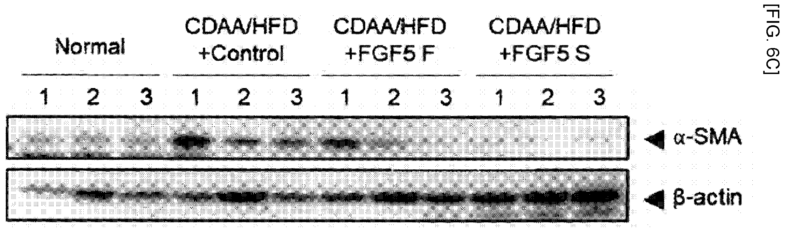

FIG. 6C shows the results of analyzing expression levels of α-SMA protein through Western blotting using the liver tissues of NAFLD mouse models after injecting the gene of FGF5-F or FGF5-v2 variant (referred to as "FGF5-v2" or "FGF5-S") into NAFLD mouse models, confirming the inhibitory effects at α-SMA protein levels by the specific injection of FGF5-v2 variant (referred to as "FGF5-v2" or "FGF5-S");

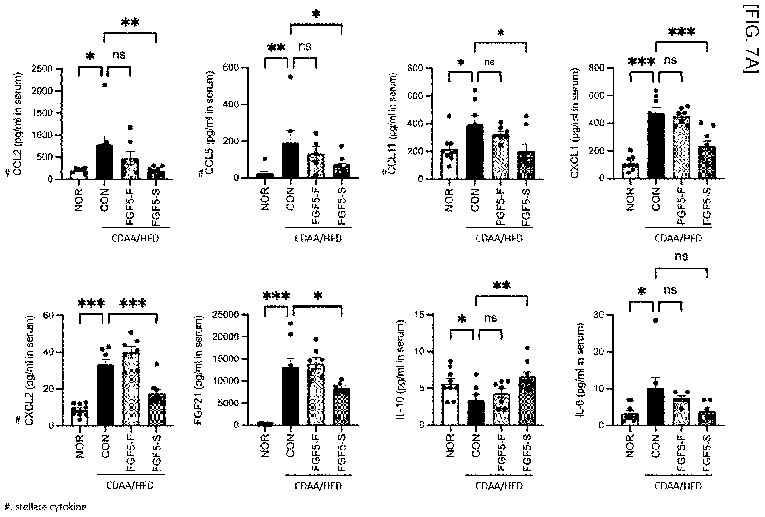

FIG. 7A shows the results of comparing expressions of various cytokines and chemokines in the sera of NAFLD mouse models after injecting the gene of FGF5-F or FGF5-v2 variant (referred to as "FGF5-v2" or "FGF5-S") into NAFLD mouse models, confirming the inhibitory effects of FGF5-v2 variant (referred to as "FGF5-v2" or "FGF5-S") on liver fibrosis;

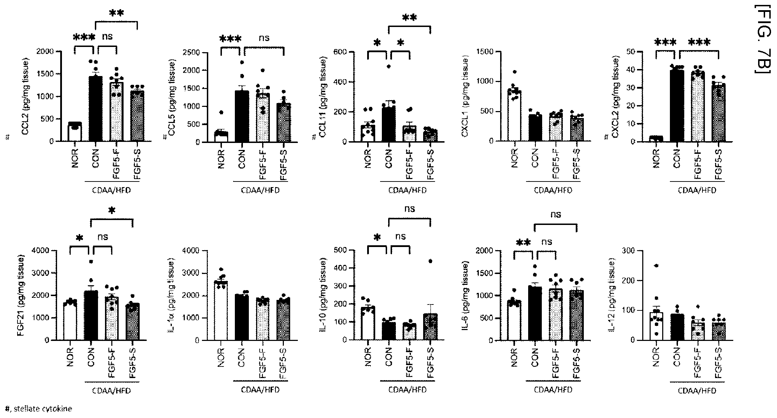

FIG. 7B shows the results of comparing expressions of various cytokines and chemokines at protein levels in the liver tissues of NAFLD mouse models after injecting the gene of FGF5-F or FGF5-v2 variant (referred to as "FGF5-v2" or "FGF5-S") into NAFLD mouse models, confirming the inhibitory effects of FGF5-v2 variant (referred to as "FGF5-v2" or "FGF5-S") on liver fibrosis-related cytokines;

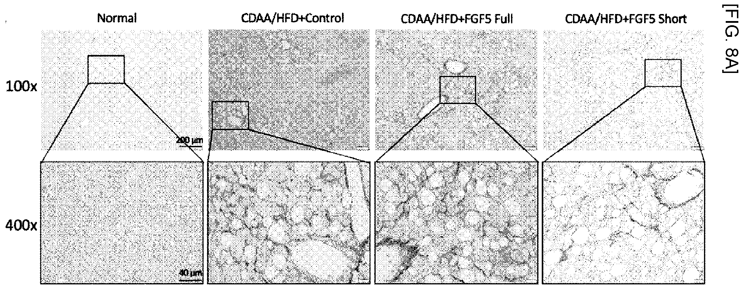

FIG. 8A shows images showing Sirius red staining results of liver tissue sections of NAFLD mouse models, into which the gene of FGF5-F or FGF5-v2 variant (referred to as "FGF5-v2" or "FGF5-S") was injected, confirming the inhibitory effects of FGF5-v2 variant (referred to as "FGF5-v2" or "FGF5-S") on liver fibrosis;

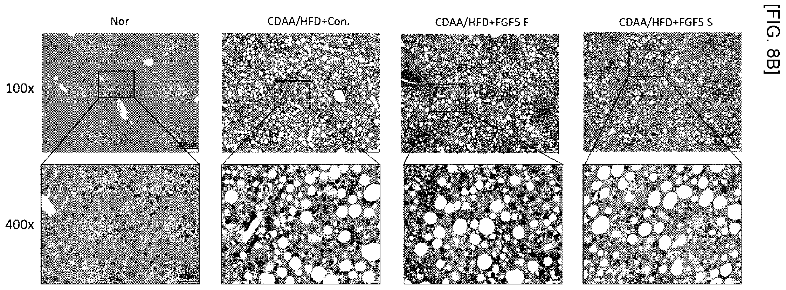

FIG. 8B shows images showing H&E staining results of liver tissue sections of NAFLD mouse models, into which the gene of FGF5-F or FGF5-v2 variant (referred to as "FGF5-v2" or "FGF5-S") was injected;

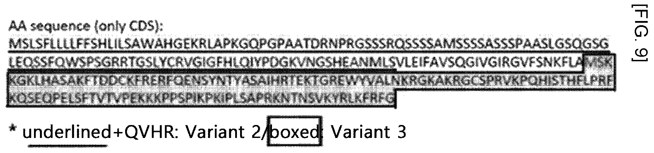

FIG. 9 shows the full-length FGF5 sequence, in which the regions corresponding to FGF5-v2 variant (referred to as "FGF5-v2" or "FGF5-S") (underlined) and FGF5-v3 (boxed) are shown;

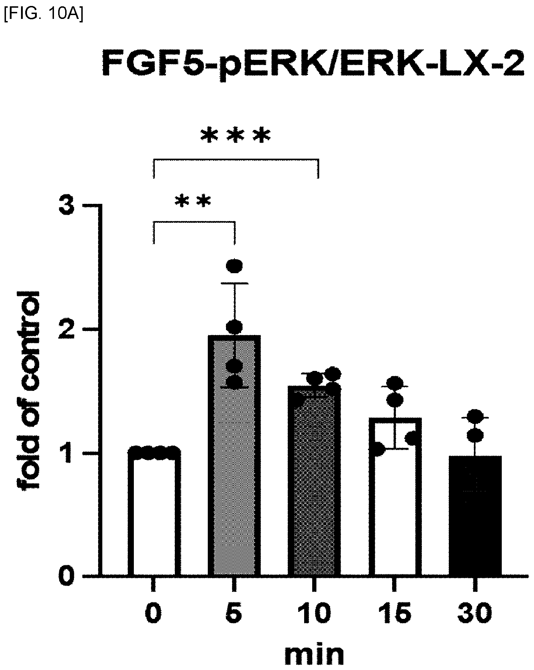

FIG. 10A shows the results of a stimulation test using a human hepatic stellate cell line (LX2 cells), in which the x-axis represents the time (min) after treatment with FGF5 and the y-axis represents the fold value of a control experiment, confirming through Erk phosphorylation reaction that the FGF5 full-length protein has a signaling effect;

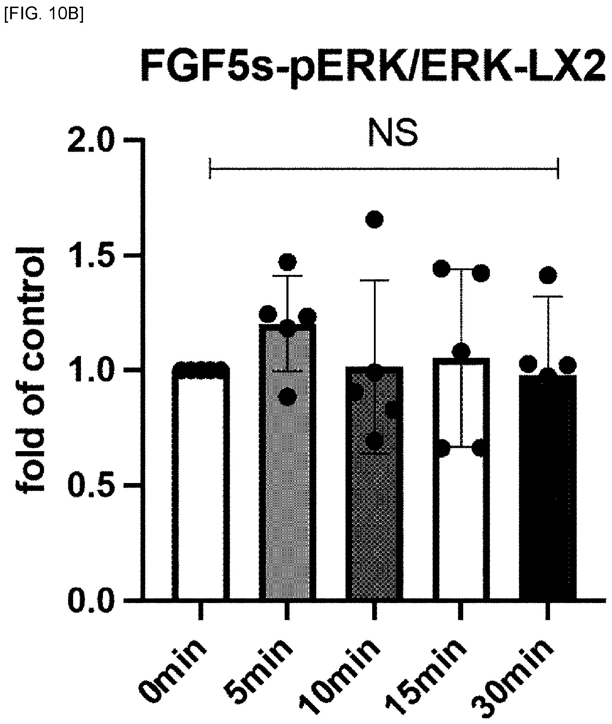

FIG. 10B shows the results of a stimulation test using a human hepatic stellate cell line (LX2 cells), in which the x-axis represents the time (min) after treatment with FGF5-v2 variant (referred to as "FGF5-v2" or "FGF5-S") protein and the y-axis represents the fold value of a control experiment, confirming through Erk phosphorylation reaction that the FGF5-v2 variant (referred to as "FGF5-v2" or "FGF5-S") protein has no signaling effect; and

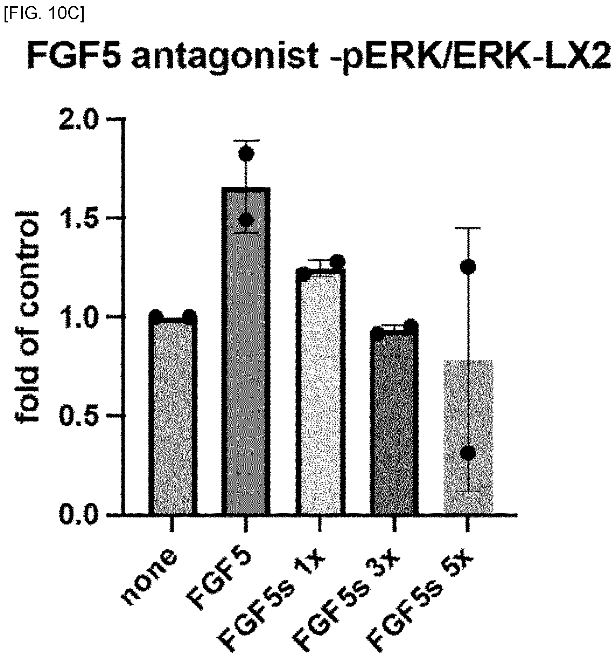

FIG. 10C shows the results of a stimulation test using a human hepatic stellate cell line (LX2 cells), in which the x-axis represents 1-, 3-, and 5-fold pretreatment with FGF5-v2 variant (referred to as "FGF5-v2" or "FGF5-S") protein for FGF5 treatment, and the y-axis represents the fold value of a control experiment, confirming that FGF5-v2 variant (referred to as "FGF5-v2" or "FGF5-S") protein antagonizes the signaling effect of FGF5 full-length protein, indicating the inhibitory effects of FGF5-v2 variant (referred to as "FGF5-v2" or "FGF5-S") on liver fibrosis.

### [Detailed Description of Preferred Embodiments]

Unless defined otherwise, all technical and scientific terms used herein have the same meanings as those generally understood by one of ordinary skill in the art.

As used herein, the term 'protein' is used interchangeably with 'polypeptide', and refers to, for example, a polymer of amino acid residues, as is generally found in proteins in their natural state.

The present disclosure provides a mutant gene of fibroblast growth factor 5 (FGF5), an FGF5 transcript variant expressed therefrom, and a protein translated therefrom. In other words, the present disclosure aims to provide a gene of FGF5 transcript variant 2 (FGF5-v2), which is an FGF5 fragment newly identified by the present inventors, a transcript expressed therefrom, and a protein translated therefrom, etc.

The FGF5-v2 variant of the present disclosure is an FGF5 fragment newly identified by the present inventors, and may be referred to as "FGF5-v2" or "FGF5-S". The present inventors have newly identified that the FGF5-v2 protein not only has different characteristics from the full-length FGF5 protein or other fragments (FGF5-v1 or FGF5-v3) but also has the effects of improving and treating liver fibrosis, which are completely different from the parent protein FGF5. The full-length FGF5 is a known protein, and specific information may be found in public databases, such as NCBI (Registration number NM_004464.4).

The FGF5-v2 protein of the present disclosure is characterized by comprising an amino acid sequence of SEQ ID NO: 1 in the sequence listing, or by consisting of the amino acid sequence. In the present disclosure, the FGF5-v2 protein is meant to comprise a functional equivalent thereof. The functional equivalent refers to a polypeptide having at least 50% or more, 70% or more, preferably 80% or more, more preferably 90% or more sequence homology (i.e., identity) to the amino acid sequence constituting the FGF5-v2 protein (for a preferred example, the amino acid sequence represented by SEQ ID NO: 1 in the sequence listing). For example, the functional equivalent comprises polypeptides having 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% sequence homology, and refers to a polypeptide exhibiting substantially the same physiological activity as the parent FGF5-v2 protein (for a preferred example, the polypeptide represented by SEQ ID NO: 1 in the sequence listing). Herein, 'substantially the same physiological activity' preferably means the effects of preventing, improving, or treating liver fibrosis. Preferably, the functional equivalent of the FGF5-v2 protein in the present disclosure may be those produced as a result of addition, substitution, or deletion of some of the amino acid sequence of SEQ ID NO: 1 in the sequence listing. The substitution of the amino acid is preferably a conservative substitution. Examples of conservative substitutions of amino acids present in nature are as follows; aliphatic amino acids (Gly, Ala, Pro), hydrophobic amino acids (Ile, Leu, Val), aromatic amino acids (Phe, Tyr, Trp), acidic amino acids (Asp, Glu), basic amino acids (His, Lys, Arg, Gln, Asn) and sulfur-containing amino acids (Cys, Met). Further, the functional equivalent of the FGF5-v2 protein comprises variants in which a portion of the amino acids is deleted in the amino acid sequence of the FGF5-v2 protein (i.e., a shorter functional fragment). The deletion or substitution of the amino acids may be preferably located in a region that is not directly related to the physiological activity of the FGF5-v2 protein. In other words, the deletion or substitution may be performed within a range that does not affect the physiological activity of FGF5-v2 protein. Furthermore, the functional equivalent comprises variants in which some amino acids are added at both ends of or in the amino acid sequence of the FGF5-v2 protein. Further, the scope of the functional equivalent of the present disclosure comprises polypeptide derivatives in which some of the chemical structures of the polypeptide are modified while maintaining the basic skeleton of the FGF5-v2 protein and physiological activity thereof. For example, structural modifications to alter the stability, storage, volatility or solubility of the protein may be comprised therein.

In the present disclosure, sequence homology and identity are defined as the percentage of identical matched residues (amino acid residues or bases) of a candidate sequence relative to an original sequence after aligning the candidate sequence with the original sequence (in the case of an amino acid sequence, a preferred example is SEQ ID NO: 1 in the sequence listing, or in the case of a nucleotide sequence, a preferred example is SEQ ID NO: 2 in the sequence listing) and introducing gaps. As needed, conservative substitutions as part of sequence identity are not considered to obtain maximum percent sequence identity. Also, when homology or identity of the protein sequence is determined, the N-terminal, C-terminal or internal stretch, deletion or insertion of the amino acid sequence of FGF5-v2 protein is not interpreted as a sequence that affects identity or homology of the sequence. In addition, the sequence identity may be determined by standard methods used to compare similar regions of amino acid sequences of two polypeptides. A computer program such as BLAST or FASTA aligns two polypeptides so that each amino acid is optimally matched (along the full length sequence of one or two sequences or along the predicted region of one or two sequences). The program provides default opening penalty and default gap penalty, and provides a scoring matrix such as PAM250 (Standard Scoring Matrix; Dayhoff et al., in Atlas of Protein Sequence and Structure, vol 5, supp 3, 1978) which may be used in conjunction with the computer program. For example, percentage identity may be calculated as follows: After multiplying the total number of identical matches by 100 and dividing by the sum of the length of a longer sequence in a matched span and the number of gaps that are introduced into the longer sequence to align the two sequences.

Further, the present disclosure provides an FGF5-v2 gene encoding the FGF5-v2 protein of the present disclosure.

As used herein, the term 'nucleic acid', 'gene sequence', 'DNA sequence', 'RNA sequence', or 'polynucleotide' refers to a deoxyribonucleotide or a ribonucleotide in a single-stranded or double-stranded form. Unless otherwise limited, it also comprises known analogs of natural nucleotides that hybridize to nucleic acids in a manner similar to naturally occurring nucleotides.

In one embodiment of the present disclosure, the FGF5-v2 gene may be characterized by comprising a nucleotide sequence of SEQ ID NO: 2 in the sequence listing. In another embodiment of the present disclosure, the FGF5-v2 gene may consist of the nucleotide sequence of SEQ ID NO: 2 in the sequence listing.

In the present disclosure, the polynucleotide encoding the expression target protein may undergo various modifications in the coding region within the scope that does not change the amino acid sequence of the protein expressed from the coding region, and various modifications in a region, other than the coding region, within the scope that does not affect expression of the gene, due to codon degeneracy or in consideration of the codons preferred in an organism in which the proteins are to be expressed. Those skilled in the art will readily understand that such modified genes also fall within the scope of the present disclosure. In other words, the polynucleotide of the present disclosure may be mutated by substitution, deletion, or insertion of one or more nucleic acid bases, or a combination thereof, as long as it encodes a protein having activity equivalent thereto, and these also fall within the scope of the present disclosure.

In other words, a nucleic acid molecule (gene) represented by a specific sequence in this specification may comprise not only the corresponding sequence but also a biological equivalent thereof. That is, considering a mutation having biologically equivalent activity to a nucleic acid molecule, the nucleic acid molecule of one aspect is interpreted to also comprise a sequence showing substantial identity to the sequence represented by the sequence number in the sequence listings. Specifically, a nucleic acid molecule comprising a nucleotide sequence represented by a specific sequence number is not limited to the corresponding nucleotide sequence, and a variant of the nucleotide sequence is comprised within the scope of the present disclosure. A nucleic acid molecule consisting of a nucleotide sequence represented by a specific sequence number in the sequence listing of the present disclosure is a concept comprising a functional equivalent of the nucleic acid molecule constituting the same, for example, a variant that has some nucleotide sequence of the nucleic acid molecule altered by deletion, substitution, or insertion, but may perform the same function as the corresponding nucleic acid molecule. Specifically, the nucleic acid molecule disclosed in the present disclosure may comprise a nucleotide sequence having 70% or more, more preferably 80% or more, much more preferably 90% or more, most preferably 95% or more sequence homology to an amino acid sequence represented by a specific sequence number in the sequence listing. For example, the nucleic acid molecule comprises a nucleic acid molecule having 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% sequence homology thereto. "% of sequence homology" to a nucleic acid molecule is confirmed by comparing two optimally aligned sequence within a comparison region, and a portion of the nucleotide sequence in the comparison region may comprise an addition or deletion (i.e., a gap) compared to the reference sequence (without addition or deletion) for the optimal alignment of the two sequences.

The gene (nucleic acid molecule) of the FGF5-v2 variant of the present disclosure, the transcript thereof, and/or the protein thereof may be delivered to an individual via various carriers or directly as it is. Any carrier is sufficient as long as it delivers the gene of the FGF5-v2, the transcript thereof, and/or the protein thereof to a host cell or other subject so that the gene is expressed in the cell or the transcript or protein exhibits its original physiological activity (inhibitory activity on liver fibrosis), and is not limited to a specific type. As the carrier for delivery of the FGF5-v2 variant, for example, expression vectors, viruses (viral particles), vesicles, nanoparticles, microparticles, liposomes, transposons, micelles, antibodies, or exosomes may be used, but are not limited thereto, and methods of delivering genes, transcripts, and proteins known in the art may be applied without limitation. In addition, the protein may be delivered directly together with a preservation material.

For example, the present disclosure provides an expression vector (recombinant expression vector) comprising the FGF5-v2 gene (i.e., nucleic acid molecule encoding the FGF5-v2 protein) according to the present disclosure. In other words, the present disclosure provides an expression vector (recombinant expression vector) comprising a promoter; and the FGF5-v2 gene (FGF5-v2 polynucleotide) operably linked thereto.

As used herein, the term 'expression' means the production of proteins or nucleic acids in cells.

As used herein, the "recombinant vector" refers to a vector capable of expressing a peptide or protein encoded by foreign nucleic acids inserted in the vector, preferably, a vector prepared so as to express a target protein (in the present disclosure, FGF5-v2 protein). The "vector" refers to any vehicle for the introduction and/or transfer of bases into a host cell *in vitro, ex vivo,* or *in vivo,* and may be a replicon to which another DNA fragment may be bound to bring about the replication of the bound fragment, and the "replicon" refers to any genetic unit (e.g., a plasmid, a phage, a cosmid, a chromosome, a virus, etc.) that functions as an autonomous unit of DNA replication *in vivo,* that is, one which is capable of replication under its own control.

The vector according to the present disclosure may be a linear DNA, a plasmid DNA, or a recombinant viral vector, but is not limited thereto. The vector comprises, for example, a plasmid vector, a cosmid vector, and a viral vector such as a bacteriophage vector, an adenovirus vector, a lentivirus vector, a retrovirus vector, and an adeno-associated virus vector. Preferably, the vector is an adenovirus vector. The present inventors used a pADTrack vector in a specific Example.

The 'promoter' refers to a DNA sequence that controls the expression of a nucleic acid sequence operably linked thereto in a specific host cell. In addition, it may further comprise any operator sequence for regulating transcription, a sequence encoding a suitable mRNA ribosome binding site, and a sequence regulating termination of transcription and translation. As the promoter, a promoter (constitutive promoter) constantly inducing expression of a target gene at all times or a promoter (inducible promoter) inducing expression of the target gene at a specific site or time may be used, and examples thereof comprise SV40 promoter, CMV promoter, CAG promoter, CaMV 35S promoter, Rsyn7 promoter (US Patent No. 08/991,601), rice actin promoter, ubiquitin promoter, ALS promoter (US Patent No. 08/409,297), etc. In addition, promoters disclosed in US Patent NOs. 5,608,149; 5,608,144, 5,604,121, 5,569,597, 5,466,785, 5,399,680, 5,268,463, 5,608,142, etc. may be all used.

The recombinant vector of the present disclosure may comprise, in addition to the promoter, any operator sequence for regulating transcription, a sequence encoding a suitable mRNA ribosome binding site, and a sequence regulating termination of transcription and translation, a terminator, etc., and more preferably, may further comprise a polyhistidine tag (an amino acid motif consisting of at least 5 or more histidine residues), a signal peptide gene, an endoplasmic reticulum retention signal peptide, a cloning site, etc., and may further comprise a tag gene, a selection marker gene such as an antibiotic resistance gene for selecting a transformant, etc. In the recombinant vector, the polynucleotide sequence of each gene is operably linked to a promoter. As used herein, the term "operably linked" means a functional linkage between a nucleotide expression regulatory sequence, such as a promoter sequence, and another nucleotide sequence, whereby the regulatory sequence regulates transcription and/or translation of another nucleotide sequence.

The recombinant vector may be constructed for use in prokaryotic or eukaryotic host cells. For example, when the vector of the present disclosure is an expression vector, and a prokaryotic cell is used as a host, the vector generally comprises a strong promoter capable of initiating transcription (e.g., pLλ promoter, trp promoter, lac promoter, tac promoter, T7 promoter, etc.), a ribosome binding site for initiating translation, and a transcription/translation termination sequence. When a eukaryotic cell is used as a host, the vector generally comprises the origin of replication acting in the eukaryotic cell, e.g., f1 origin of replication, SV40 origin of replication, pMB1 origin of replication, adeno origin of replication, AAV origin of replication, or BBV origin of replication, etc., but is not limited thereto. In addition, a promoter derived from the genomes of mammalian cells (e.g., a metallothionein promoter) or a promoter derived from mammalian viruses (e.g., an adenovirus late promoter, a Vaccinia virus 7.5K promoter, an SV40 promoter, a cytomegalovirus promoter, and a tk promoter of HSV) may be used. As a transcription termination sequence, a polyadenylation sequence may generally be used.

The tag gene may be represented by an Avi tag, a Calmodulin tag, a polyglutamate tag, an E tag, a FLAG tag, an HA tag, a His tag (polyhistidine tag), a Myc tag, an S tag, an SBP tag, an IgG-Fc tag, a CTB tag, a Softag 1 tag, a Softag 3 tag, a Strep tag, a TC tag, a V5 tag, a VSV tag, an Xpress tag, etc. Preferably, the vector according to the present disclosure may comprise a myc tag.

Meanwhile, the expression vector may be introduced into a target cell (host cell) in an expression form by a method known in the art, such as infection, transfection, or transduction, etc.

A gene transfer method using a plasmid expression vector is a method of directly transferring a plasmid DNA to mammalian cells, and is a method approved for use in humans by the FDA. The plasmid DNA has the advantage of being homogeneously purified, unlike viral vectors. As the plasmid expression vector that may be used in the present disclosure, mammalian expression plasmids known in the art may be used. Representative examples comprise, but are not limited to, pRK5 (European Patent No. 307,247), pSV16B (International Patent Publication No. 91/08291) and pVL1392 (PharMingen), etc. The plasmid expression vector may be introduced into target cells by a method known in the art, for example, but not limited to, transient transfection, microinjection, transduction, cell fusion, calcium phosphate precipitation, liposome-mediated transfection, DEAE Dextran-mediated transfection, polybrene-mediated transfection, electroporation, gene gun, and other known methods of introducing DNA into cells.

Further, with regard to the method applicable to the present disclosure, viral expression vectors comprising the nucleic acids comprise, but are not limited to, retrovirus, adenovirus, herpes virus, avipox virus, lentivirus, etc. The retroviral vector is constructed (produced) such that viral genes are all removed or modified to produce non-viral proteins in cells infected with the viral vector. The main advantages of the retroviral vectors for gene therapy are that they deliver large amounts of genes into replicative cells, precisely integrate the transferred genes into cellular DNA, and do not induce continuous infections after gene transfection. The retroviral vector approved by the FDA is those prepared using PA317 amphotropic retrovirus packaging cells. Non-retroviral vectors comprise adenovirus as described above. The main advantages of adenovirus are that it transfers large amounts of DNA fragments (36 kb genomes) and is capable of infecting non-replicative cells at a very high titer. Moreover, herpes virus may also be useful for human genetic therapy. In addition, any suitable viral vector known in the art may be used in the present disclosure.

When the expression vector is a viral recombinant vector, the vector may be transfected into a virus producing cell, i.e., a packaging cell line. Any of a variety of techniques commonly used to introduce exogenous nucleic acids (DNA or RNA) into prokaryotic or eukaryotic host cells for "transfection" may be used, for example, electrophoresis, calcium phosphate precipitation, DEAE-dextran transfection, or lipofection. A virus comprising the target gene (FGF5-v2 gene) according to the present disclosure may be propagated within the packaging cell line and released outside the cell, and the virus may be transduced into target cells. The nucleic acid of the virus "transduced" into the cells is used to produce the target transcript or protein (FGF5-v2 transcript or protein) with or without being inserted into the genome of the cell.

In addition, the present disclosure provides is an isolated cell into which the expression vector according to the present disclosure has been introduced (transformed, transfected, transduced, etc.). The cell refers to a cell for proliferating (amplifying) the expression vector. That is, the cell refers to a host cell into which the above-described nucleic acid molecule or expression vector is directly transduced/transformed/transfected. For example, when the expression vector is a viral vector, the cell may be a packaging cell for producing a virus comprising the viral vector. The selection of a suitable host is considered to be apparent to those skilled in the art from the teachings of the present disclosure.

To achieve another object of the present disclosure, the present disclosure provides a pharmaceutical composition for preventing or treating liver fibrosis (liver fibrotic disease), the pharmaceutical composition comprising, as an active ingredient, the FGF5-v2 transcript according to the present disclosure, the FGF5-v2 protein, or the expression vector comprising the nucleic acid molecule encoding the FGF5-v2 protein; and/or the isolated cell, into which the expression vector is introduced. In addition to the components mentioned above, the pharmaceutical composition may comprise various carriers for delivering the FGF5-v2 gene, transcript, and/or protein of the present disclosure to target cells. In other words, the pharmaceutical composition may comprise the FGF5-v2 gene, transcript, and/or protein which are/is loaded on the carrier. A detailed description of the carrier will be omitted as it has been described above.

As used herein, "liver fibrosis (liver fibrotic disease)" refers to a disease in which the liver is damaged in its shape or function by the accumulation of fibrosis due to continuous damage to liver tissue by various causes. The term "liver fibrosis" may be used interchangeably with "liver fibrotic disease". The fibrosis is a phenomenon resulting from excessive formation of fibrous connective tissue in organs or tissues during the wound recovery process due to repeated damage to cells or tissues. Since persistent accumulation of liver fibrosis causes various liver diseases comprising liver cancer and cirrhosis, etc., the development of serious diseases may be prevented or liver dysfunction may be treated by suppressing and improving liver fibrosis. The present inventors confirmed that FGF5-v2 of the present disclosure effectively suppresses fibrosis in the liver tissue of a liver disease animal model and suppresses the expression of cytokines related to fibrosis and inflammation, etc., thereby improving the disease.

In one embodiment of the present disclosure, the liver fibrosis comprises alcoholic fatty liver disease, non-alcoholic fatty liver disease, hepatitis C, hepatitis B, autoimmune hepatitis, hepatic encephalopathy, primary biliary cirrhosis, liver cancer, and hepatocellular carcinoma, as well as unknown or unspecified liver fibrosis and other chronic liver diseases, etc., but is not limited thereto, and any disease may be comprised without limitation as long as it is directly or indirectly caused by liver fibrosis or accompanied by liver fibrosis.

In one embodiment of the present disclosure, the non-alcoholic fatty liver disease may be any one or more selected from the group consisting of non-alcoholic steatohepatitis (NASH), non-alcoholic fatty liver (NAFL), hepatic steatosis, acute fatty liver of pregnancy (AFLP), NAFLD-associated liver failure, NAFLD-associated liver fibrosis, NAFLD-associated hepatocirrhosis, and NAFLD-associated liver cancer, but is not limited thereto.

In another embodiment of the present disclosure, the pharmaceutical composition (i.e., FGF5-v2 protein or expression vector thereof, etc.) of the present disclosure may be characterized by being co-administered with an anti-inflammatory agent, but is not limited thereto. In other words, when the FGF5-v2 of the present disclosure is co-administered with existing anti-inflammatory agents, a better anti-fibrotic effect may be achieved.

As used herein, the term "co-administration" may refer to the simultaneous, sequential, or separate administration of the individual components of a treatment regimen. Combination therapy may be defined as achieving a combined therapeutic effect by administering two or more drugs simultaneously or sequentially, or alternately at regular or undetermined intervals, such that the efficacy, as measured, for example, by degree of response, rate of response, duration of disease progression, or survival period, is therapeutically superior to the efficacy achieved by administering one or the other of the components of the combination therapy at usual doses, while providing synergistic effects, but is not limited thereto.

The pharmaceutical composition of the present disclosure may be administered simultaneously, separately, or sequentially with an anti-inflammatory agent. Even when administered sequentially with the anti-inflammatory agent, there is no limitation on the order of administration. However, the dosage regimen may be appropriately adjusted depending on the type of anti-inflammatory agent, a patient's conditions, sex, age, etc.

Further, the present disclosure provides a pharmaceutical composition for preventing or treating liver fibrosis, the pharmaceutical composition comprising, as an active ingredient, the FGF5-v2 transcript according to the present disclosure, (i) the FGF5-v2 protein of the present disclosure, the expression vector comprising the nucleic acid molecule encoding the same, or the isolated cell, into which the expression vector is introduced; and (ii) an anti-inflammatory agent.

The composition may be in the form of a mixture in which the (i) FGF5-v2 protein, etc., and (ii) the anti-inflammatory agent are mixed, for simultaneous administration of the (i) FGF5-v2 protein, etc., and (ii) the anti-inflammatory agent. For example, the (i) FGF5-v2 protein, etc., and (ii) the anti-inflammatory agent may be in a single formulation prepared by mixing with a pharmaceutically acceptable adjuvant, diluent, or carrier. At this time, the (i) FGF5-v2 protein, etc. and (ii) the anti-inflammatory agent may each comprise multiple ingredients within the category of the corresponding drug. In addition, the single formulation may be administered together with a separate formulation comprising another therapeutic agent as a formulation separately from this formulation.

Alternatively, the composition may be in a form in which (i) the FGF5-v2 protein, etc., and (ii) the anti-inflammatory agent are each formulated and administered simultaneously, separately, or sequentially. In this case, the composition may be a pharmaceutical composition for combined administration for simultaneous or sequential administration, the pharmaceutical composition comprising a first pharmaceutical composition comprising a pharmaceutically effective amount of (i) FGF5-v2 protein, etc. as an active ingredient; and a second pharmaceutical composition comprising a pharmaceutically effective amount of (ii) the anti-inflammatory agent as an active ingredient.

The anti-inflammatory agent according to the present disclosure may be a steroidal anti-inflammatory agent and/or a non-steroidal anti-inflammatory agent. The non-steroidal anti-inflammatory agent may be selected from salicylic acid, acetic acid, propionic acid, mefenamic acid, oxicam series or non-acidic series, and specific examples comprise, but are not limited to, aspirin, ibuprofen, naproxen, ketoprofen, and fenoprofen.

Further, the present disclosure provides a kit for preventing or treating liver fibrosis, the kit comprising the pharmaceutical composition according to the present disclosure. A specific configuration of the kit according to the present disclosure is not limited, as long as it is to prevent or treat liver fibrosis, and the kit may comprise any component and device for preparing, storing, expressing, or administering the FGF5-v2 gene, transcript, protein, and expression vector according to the present disclosure, etc. without limitation.

The content of the FGF5-v2 gene, transcript thereof, protein thereof, and/or expression vector, etc. in the composition of the present disclosure may be appropriately adjusted depending on the symptoms of the disease, the degree of progression of the symptoms, the conditions of the patient, etc., for example, 0.0001% by weight to 99.9% by weight, or 0.001% by weight to 50% by weight, based on the total weight of the composition, but is not limited thereto. The content ratio is a value, based on the dry amount from which the solvent is removed.

The pharmaceutical composition according to the present disclosure may further comprise suitable carriers, excipients, and diluents that are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled-release additive.

The pharmaceutical composition according to the present disclosure may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

The carrier, the excipient, and the diluent that may be included in the pharmaceutical composition according to the present disclosure may comprise lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

For formulation, preparation is performed using commonly used diluents or excipients, such as fillers, thickeners, binders, wetting agents, disintegrants, surfactants, etc.

As additives for tablets, powders, granules, capsules, pills, and troches according to the present disclosure, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC) 1928, HPMC 2208, HPMC 2906, HPMC 2910, propylene glycol, casein, calcium lactate, Primojel, etc.; binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, polyvinylpyrrolidone, etc. may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, light anhydrous silicic acid, etc.; lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, light anhydrous silicic acid, etc. may be used.

As additives for liquids according to the present disclosure, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present disclosure, a white sugar solution, other sugars or sweeteners, etc. may be used, and as needed, a fragrance, a colorant, a preservative, a stabilizer, a suspending agent, an emulsifier, a viscous agent, etc. may be used.

In emulsions according to the present disclosure, purified water may be used, and as needed, an emulsifier, a preservative, a stabilizer, a fragrance, etc. may be used.

In suspensions according to the present disclosure, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, etc. may be used, and as needed, a surfactant, a preservative, a stabilizer, a colorant, and a fragrance may be used.

Injections according to the present disclosure may comprise solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; pain relief agents such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present disclosure, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan (propylene glycol monostearate), glycerin, Adeps solidus, buytyrum Tego-G, cebes Pharma 16, hexalide base 95, Cotomar, Hydrokote SP, S-70-XXA, S-70-XX75(S-70-XX95), Hydrokote 25, Hydrokote 711, Idropostal, Massa estrarium (A, AS, B, C, D, E, I, T), masa-MF, masupol, masupol-15, neosuppostal-N, paramount-B, supposiro (OSI, OSIX, A, B, C, D, H, L), suppository base IV types (AB, B, A, BC, BBG, E, BGF, C, D, 299), suppostal (N, Es), Wecoby (W, R, S, M, Fs), and tegester triglyceride matter (TG-95, MA, 57) may be used.

Solid preparations for oral administration comprise tablets, pills, powders, granules, capsules, etc., and such solid preparations are formulated by mixing the extract with at least one or more excipient, e.g., starch, calcium carbonate, sucrose, lactose, gelatin, etc. In addition to simple excipients, lubricants such as magnesium stearate and talc are also used.

Liquid preparations for oral administration comprise suspensions, liquids for internal use, emulsions, syrups, etc., and these liquid preparations may comprise, in addition to simple commonly used diluents, such as water and liquid paraffin, various types of excipients, for example, a wetting agent, a sweetener, a fragrance, a preservative, etc. Preparations for parenteral administration comprise an aqueous sterile solution, a non-aqueous solvent, a suspension, an emulsion, a freeze-dried preparation, and a suppository. As the non-aqueous solvent and the suspension, propylene glycol, polyethylene glycol, a vegetable oil such as olive oil, and an injectable ester such as ethyl oleate, etc. may be used.

The pharmaceutical composition according to the present disclosure is administered in a pharmaceutically effective amount. In the present disclosure, the "pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The pharmaceutical composition according to the present disclosure may be administered as an individual therapeutic agent, may be administered in combination with other therapeutic agents, may be administered sequentially or simultaneously with traditional therapeutic agents, and may be administered once or multiple times. In consideration of all the above factors, it is important to administer an amount capable of obtaining the maximum effect with a minimum amount without side effects, which may easily be determined by those skilled in the art to which the present disclosure pertains.

The pharmaceutical composition of the present disclosure may be administered to an individual by various routes. All modes of administration may be considered, for example, oral administration, subcutaneous injection, intraperitoneal administration, intravenous injection, intramuscular injection, paraspinal space (intrathecal) injection, sublingual administration, buccal administration, rectal insertion, vaginal insertion, ocular administration, otic administration, nasal administration, inhalation, spray through the mouth or nose, dermal administration, transdermal administration, etc.

The pharmaceutical composition of the present disclosure is determined according to the type of drug as an active ingredient along with several related factors, e.g., the disease to be treated, route of administration, patient's age, sex, body weight, and severity of the disease. Specifically, the effective amount of the composition according to the present disclosure may vary depending on the patient's age, sex, and body weight, and the composition may be generally administered in an amount of 0.001 mg to 150 mg, preferably 0.01 mg to 100 mg per 1 kg of body weight, daily or every other day, or divided into once to three times a day. However, since the dosage may increase or decrease depending on the route of administration, severity of the disease, sex, body weight, age, etc., the above dosage does not limit the scope of the present disclosure in any way.

As used herein, the term "individual" refers to a subject in need of treatment for a disease, and more specifically, a mammal of humans or non-human primates, mice, rats, dogs, cats, horses, cattle, etc.

As used herein, the term "administration" refers to provision of a predetermined composition of the present disclosure to an individual by any suitable method.

As used herein, the term "preventing" refers to all actions that inhibit or delay the onset of a target disease, the term "treating" refers to all actions that improve or beneficially change a target disease and metabolic abnormalities thereof by the administration of the pharmaceutical composition according to the present disclosure, and the term "improving" refers to all actions that reduce target disease-related parameters, for example, the degree of symptoms by the administration of the composition according to the present disclosure.

### [Mode for Carrying Out the Invention]

Hereinafter, preferred Examples and Experimental Examples are presented to help the understanding of the present disclosure. However, the following Examples and Experimental Examples are provided only to facilitate easier understanding of the present disclosure, and the content of the present disclosure is not limited by the following Examples and Experimental Examples.

### Example 1. Production of FGF5-v2 (FGF5 transcript variant 2) and Preparation of gene injection

pAdTrack-CMV vector was digested using restriction enzymes KpnI and HindII, and a gene was synthesized using the PN 5'-AAAGGTACCATGAGCTTGTCCTTCCTCCTC-3' primer (SEQ ID NO: 19 in the sequence listing) and 5'-AAAAAGCTTTCACTTATCGTCGTCATCCTTGTAATCTCTGTGAACTTGGCTTAA CATATTGGCTTCGT-' primer (SEQ ID NO: 20 in the sequence listing) corresponding to FGF5 variant 2, and digested with the restriction enzymes KpnI and HindII, followed by ligation. The prepared recombinant vector (pAdTrack-CMV hFGF5-2) was transformed into *E*. *coli* to be amplified. The pAdTrack-CMV hFGF5-2 vector and pAdEASY vector digested with Pmel restriction enzyme were transformed into *E*. *coli* BJ5183 cells, and the transformed *E*. *coli* BJ5183 cells-hFGF5-2 expressing hFGF5-2 were selected. Thereafter, the target gene was isolated, and adenovirus hFGF5-2 was prepared using 293T cells.

### Example 2. Preparation of in vivo diet-induced non-alcoholic fatty liver disease (hereinbelow, NAFLD) as liver fibrosis model and Gene injection

Specific pathogen-free male C57BL/6J mice (6-week-old) were purchased from DBL (Chungcheong-do, Korea) and acclimated to the feeding environment for 1 week before the start of treatment. At 7 weeks of age, the mice were randomly divided into two groups. For 6 weeks, a normal group (n=8) was fed a commercial standard diet, and a liver fibrosis group (n=24) was fed CDAA-HFD (A06071302, Research Diets, NJ, USA). CDAA-HFD is a choline-deficient high-fat diet containing L-amino acid and methionine, which induces NAFLD (nonalcoholic fatty liver disease; simple fatty liver, steatohepatitis, and liver fibrosis (nonalcoholic fatty liver-associated cirrhosis).

The liver fibrosis group was again divided into an empty vector administration group; a full-length FGF5 (also referred to as FGF5-F; FGF5 full sequence) administration group; and an FGF5 variant FGF5-v2 (also referred to as FGF5-S) administration group, and the experiments were conducted. To this end, 1 × 10⁹ of the pAd-GFP-control group (n=8), pAd-hFGF5 (vector for FGF5-F expression) (n=8), or pAd-hFGF5-v2 (vector for FGF5-v2 expression) (n=8) prepared in Example 1 were injected at a time through the tail vein of each mouse. PBS was administered to the normal mouse group that was not fed the high-fat diet. 12 days later, a glucose tolerance test was performed on all mice, and 2 days later, the animals were euthanized with CO₂. All mice were weighed weekly. The mice were housed in a room with controlled temperature (23 ± 3°C) and relative humidity (40% to 60%) under specific pathogen-free conditions.

### Example 3.: Investigation of inhibitory effect of FGF5-v2 gene injection on liver fibrosis in in vivo NAFLD mouse model

As described above, normal mice or NAFLD mice were treated with the empty vector (control), full-length FGF5 (FGF5-F), or FGF5-v2 (FGF5-S), and after performing a glucose tolerance test (GTT), the liver tissues of each mouse were collected, and the NAS score, which is a steatosis index, and fibrosis score were measured. In detail, the degrees of steatosis and fibrosis in the tissues were scored by pathologists based on the photographs of the stained mouse tissues. Steatosis was numerically scored (steatosis, inflammation, and hepatocyte expansion) according to quantitative pathological standards. Fibrosis was evaluated by observing the liver tissue sections of the mice after Sirius Red staining, and scoring the same as F0 (no fibrosis), F1 (mild), F2 (moderate), F3 (bridging fibrosis), and F4 (cirrhosis).

First, the results of the steatosis evaluation are shown in FIG. 3A. The NAS score, which is a steatosis index, was significantly increased in the CDAA-HFD-fed NAFLD mice, as compared to the normal diet-fed mice, and there was no significant difference in the NAS score between the untreated control group, FGF5-F treated group, and FGF5-S treated group among the NAFLD mice. In contrast, as a result of comparing the degree of fibrosis, as confirmed in FIG. 3B, the degree of fibrosis, which was significantly increased by the CDAA-HFD diet, was not significantly different from the untreated control group, when full-length FGF5-F was treated, whereas the degree of fibrosis was significantly reduced, when FGF5-v2, which is the FGF5 transcript variant of the present disclosure, was treated. These results indicate that FGF5-v2 had the specific remarkable improvement effect on liver fibrosis appearing in fatty liver rather than on fatty liver itself.

### Example 4. Investigation of inhibition of non-alcoholic fatty liver fibrosis in serum of in vivo NAFLD mouse model by FGF5-v2 gene injection

In this Example, the inhibitory effect of the FGF5 transcript variant of the present disclosure on liver fibrosis was investigated through serum analysis of mouse models. Specifically, the serum levels of biomarkers indicating the degree of fatty liver fibrosis were quantified.

In detail, mice were anesthetized with isoflurane using a respiratory anesthetic machine, and then euthanized, and blood was collected by cardiac puncture. The collected blood was centrifuged at 4°C, 4,000 rpm for 30 minutes, and the serum was collected and stored at -80°C for use in later analysis. The levels of all metabolic factors (AST, ALT, TG, HDL, LDL, and glucose, etc.) present in the serum were measured using the Advia 1800 Chemistry System according to the manufacturer's protocol (Siemens Healthcare, Erlangen, Germany) in the serum samples obtained as described above.

As a result, there was no significant difference in HDL and LDL between the untreated control group (Control) and the full-length FGF5 or FGF5-v2 treated group (FIGS. 4E and 4F). However, the results of analyzing AST and ALT, which are used as hepatocellular damage markers in fatty liver disease, showed that the levels thereof were significantly reduced in the FGF5-v2 treated group, as compared to the untreated control group or the full-length FGF5 treated group (FIGS. 4A to 4C). It was confirmed that triglyceride (TG) and glucose were also significantly reduced in the FGF5-v2 treated group, as compared to the other groups (FIGS. 4D and 4G). These results demonstrate that FGF5-v2 of the present disclosure has a particularly excellent inhibitory effect on liver fibrosis in non-alcoholic fatty liver associated diseases.

### Example 5. Investigation of inhibition of liver fibrosis markers in liver tissue mRNA of in vivo NAFLD mouse model by FGF5-v2 gene injection

In this Example, the mRNA levels of fibrosis-related factors in the liver tissue of each mouse model were analyzed to investigate the inhibitory effect of the FGF5 transcript variant of the present disclosure on liver fibrosis at the RNA level.

RNA was isolated using the RNeasy Mini Kit (QIAGEN, Hilden, Germany) according to the manufacturer's protocol. RNA quality and total RNA yield, 260/280 and 260/230 ratios were measured using a NanoDrop spectrophotometer (Thermo, MA, USA). Total RNA was reverse-transcribed into complementary DNA using the PrimeScript^{™} 1st strand cDNA Synthesis Kit according to the manufacturer's protocol (Takara, Shiga, Japan). Quantification of the complementary DNA template was performed by real-time PCR using SYBR green fluorescence (Takara) on a CFX384 instrument (Bio-Rad, CA, USA). The primer information is as follows: mα-SMA forward 5'- TGC TGA CAG AGG CAC TGAA-3' and reverse 5'- CAG TTG TAC GTC CAG AGG CAT AG-3'; mCol1α1 forward 5'-CCT CAG GGT ATT GCT GGA CAA C-3' and reverse 5'- CAG AAG GAC CTT GTT TGC CAG G-3'; mFibronectin forward 5'-CCC TAT CTC TGA TAC CGT TGT CC - 3' and reverse 5'- TGC CGC AAC TAC TGT GAT TCG G -3'; mVimentin forward 5'-CGG AAA GTG GAA TCC TTG CAG G-3' and reverse 5' AGC AGT GAG GTC AGG CTT GGAA-3'; mHPRT forward 5' CTG GTG AAA AGG ACC TCT CGA AG-3' and reverse 5'- CCA GTT TCA CTAATG ACA CAAACG-3'.

As a result, α-SMA and Col1α1, which are major markers of fibrosis, were significantly decreased in the FGF5-v2 treated group, as compared to the control group or the FGF5 (Full) treated group (FIGS. 5A and 5B). Fibronectin and Vimentin, which are also known as EMT markers, showed increased levels in the FGF5 (Full) treated group, as compared to the untreated control group, whereas the FGF5-v2 treated group showed a level similar to the control group (FIGS. 5C and 5D). The above results demonstrate that the FGF5 transcript variant of the present disclosure is able to inhibit liver fibrosis by suppressing the expression of liver fibrosis-related factors in non-alcoholic fatty liver disease. Meanwhile, as a result of measuring the transcript expression of full-length FGF5 and FGF5-v2 in each mouse model into which the full-length FGF5 or FGF5-v2 gene was introduced, it was confirmed that only the full-length FGF5 was detected without detection of the FGF5-v2 transcript in the full-length FGF5 gene injected group, whereas only FGF5-v2 was detected without detection of the full-length FGF5 transcript in the FGF5-v2 gene injected group (FIGS. 5E and 5F).

### Example 6. Investigation of inhibition of liver fibrosis in liver tissue protein level of in vivo NAFLD mouse model by FGF5-v2 gene injection

In this Example, the protein levels of fibrosis-related factors in the liver tissue of each mouse model were analyzed by ELISA and Western blotting to investigate the inhibitory effect of the FGF5 transcript variant of the present disclosure on liver fibrosis at the protein level.

In detail, liver tissues isolated from the mice were lysed in T-PER buffer (Thermo, MA, USA) supplemented with PhosSTOP and a protease inhibitor (Roche, Basel, Switzerland), and centrifuged at 4°C, 15,000 rpm for 10 min. Protein concentrations were quantified by BCA assay (Thermo). With respect to a predetermined concentration of tissue proteins, the quantities of IL-6 and IL-1β (R&D systems, MA, USA) were measured by ELISA experimental techniques, or by immunoblotting experiment using SDS-PAGE. Antibodies such as α-SMA (abcam, Cambridge, UK) and β-actin (Cell signaling, MA, USA), etc. were used.

As a result, the FGF5-v2 treated group showed no significant difference in the inflammatory markers IL-1β and IL-6, as compared to the control group (see FIGS. 6A and 6B), but the fibrosis marker α-SMA was significantly reduced in the FGF5-v2-injected mouse model, as compared to the other groups (FIG. 6C). The results imply that administration of the FGF5 transcript variant of the present disclosure is particularly effective in reducing liver fibrosis, compared to improving inflammation, in the non-alcoholic fatty liver mouse model.

### Example 7. Investigation of cytokine and chemokine protein expression in serum and liver tissue of FGF5-v2 gene-injected in vivo NAFLD mouse model

In this Example, the levels of cytokines and chemokines in the serum and liver tissue of each mouse model were analyzed to investigate the therapeutic effect according to the administration of the FGF5 transcript variant of the present disclosure.

In detail, serum was obtained from each mouse as described in Example 4, and liver tissue fractions were obtained in the same manner as the experimental method described in Example 6. The expression levels of cytokine and chemokine proteins were measured using the MILLIPLEX MAP Mouse Cytokine/Chemokine Kit (R&D systems). The fluorescence intensity was read on MAGPIX^{®} (Luminex Corporation, Austin, TX).

As a result, as shown in FIG. 7A, the levels of cytokines and chemokines (CCL2, CCL5, CCL11, and CXCL2) secreted by hepatic stellate cells, which are the main cells involved in liver fibrosis, were significantly reduced in the FGF5-v2-administered NAFLD mice, as compared to the other NAFLD mouse groups. Further, CXCL1 and fibroblast growth factor 21 (FGF21), which are involved in fibrosis, were also confirmed to be significantly reduced in the FGF5-v2-administered group. Furthermore, the serum level of IL-10, which is an anti-inflammatory cytokine, was found to be higher in the FGF5-v2 administered group than in the other groups.

As shown in FIG. 7B, the expression of CCL2, CCL5, CCL11, and CXCL2 decreased in the FGF5-v2 administered group, as compared to the other groups, at the protein level in the liver tissue, and the level of fibroblast growth factor 21 (FGF21) also significantly decreased.

The above results demonstrate that the FGF5 transcript variant of the present disclosure is able to improve and treat liver fibrosis by inhibiting the production and secretion of cytokines and chemokines, which promote fibrosis in non-alcoholic fatty liver disease, and by increasing the level of anti-inflammatory cytokines.

### Example 8. Comparison of liver tissue staining (Sirius red staining and H&E staining) of FGF5-v2 gene-injected in vivo NAFLD mouse models

In this Example, in the liver tissues of the NAFLD mouse models, the progression of liver fibrosis was investigated through Sirius red staining, which is known as a fibrosis marker, and the progression of fatty liver disease was investigated through H&E staining.

In detail, as in the previous Example, liver tissue samples were obtained from each mouse, fixed in 10% buffered formalin, embedded in paraffin, and then sectioned into 5 µm thickness to obtain tissue sections. Each tissue section was stained with Sirius red using the Picro-Sirius Red Staining Kit (abcam) according to the manufacturer's instructions. H&E staining was performed according to the usual method.

The results of Sirius red staining are shown in FIG. 8A, and the results of H&E staining are shown in FIG. 8B. The Sirius red-stained liver tissues of the tissue sections were comparatively analyzed at 100x or 400x magnification, and as a result, the liver tissues of the FGF5 full gene-injected group showed no significant difference in the degree of Sirius red staining, as compared to the untreated control group. In contrast, it was confirmed that the liver tissues of the FGF5-v2 gene-injected mice showed a significantly reduced degree of Sirius red staining, as compared to the other groups (FIG. 8A). The above results demonstrate that FGF5-v2 is able to effectively inhibit fibrosis in the liver.

As a result of comparative histopathological analysis by magnifying the H&E stained liver tissues at 100x or 400x magnification, it was confirmed that there was no significant difference in the degree of steatosis in the histopathological analysis of H&E staining between the liver tissue of the FGF5 full gene-injected group, the liver tissue of the FGF5-v2 gene-injected group, and the untreated control group (FIG. 8B). The above results demonstrate that the FGF5-v2 gene has a specific remarkable improvement effect on liver fibrosis appearing in fatty liver rather than on fatty liver itself.

### Example 9. Investigation of antagonistic action of FGF5 through hepatic stellate cell line experiment of FGF5-v2 protein

In this example, a stimulation test was performed using a hepatic stellate cell line (LX2 cells) to investigate the inhibitory effect of the FGF5 transcript variant of the present disclosure on liver fibrosis at the protein level.

Hepatic stellate cells (LX2 cells) were seeded in the same manner the day before, and the next day, starved for about 1 hour, and treated with the recombinant protein FGF5 at 100 ng/ml to examine the protein levels of total ERK and phospho-ERK at time points. At this time, hepatoblastoma (HepG2) was also experimented as a control.

As a result, the level of total ERK did not change, but the level of phospho-ERK increased in the group treated briefly for only 5 minutes, and then gradually decreased in the group treated for 10 minutes (FIG. 10A). However, there was no significant difference in the hepatoblastoma (HepG2).

In addition, when only FGF5-v2 protein (FGF5s) was treated under the same conditions as above in order to investigate the action of FGF5-v2, the results of semi-quantifying the stimulation response of the hepatic stellate cells by a phospho-ERK response showed no significant difference in the response level for the variant protein (FIG. 10B).

Further, under the same conditions, FGF5 and FGF5-v2 protein (FGF5s) were treated together at 1-fold, 3-fold, and 5-fold. As a result, when only FGF5 was treated, the phospho-ERK level increased, whereas the phospho-ERK level decreased in the FGF5-v2 treated group. When a larger amount of FGF5-v2 was treated, the phospho-ERK level decreased more than the level when the same amount was treated, indicating that FGF5-v2 has an antagonistic effect on FGF5 (FIG. 10C). There was no change in hepatoblastoma (HepG2) when performed in the same manner.

When the experimental results of the hepatic stellate cells (LX2 cells) were corrected using the semi-quantitative value of total ERK and then the expression value of phospho-ERK was examined, a significant difference was observed. This result is the same result as the mechanism inferred from the result of the animal experiment performed above, and demonstrates that the FGF5-v2 protein is effective in suppressing liver fibrosis by antagonizing the action of FGF5 on hepatic stellate cells.

As examined in the Examples above, the FGF5 transcript variant, FGF5-v2, according to the present disclosure may effectively inhibit and improve liver fibrosis by suppressing the levels of fibrosis-promoting factors and increasing the levels of anti-inflammatory cytokines in the non-alcoholic fatty liver animal models. Accordingly, FGF-v2 of the present disclosure is expected to be useful for the prevention and treatment of various liver fibrosis-related diseases, such as cirrhosis and liver cancer, through its excellent inhibitory effect on liver fibrosis.

The above description of the present disclosure is for illustrative purposes only, and a person skilled in the art to which the present invention pertains can understand that the present disclosure may be embodied in other specific forms without departing from the technical spirit or essential characteristics thereof. In this regard, the embodiments described above should be understood to be illustrative rather than restrictive in every respect.

Sequence information related to the present disclosure is shown in Table 1 below.

**[Table 1]**

| Gene | Sequence | SEQ ID NO. |
|---|---|---|
| FGF5-v2 | MSLSFLLLLFFSHLILSAWAHGEKRLAPKGQPGPAATDRNPRGS SSRQSSSSAMSSSSASSSPAASLGSQGSGLEQSSFQWSPSGR RTGSLYCRVGIGFHLQIYPDGKVNGSHEANMLSQVHR | 1 |
| FGF5-v2_only CDS | atgagcttgtccttcctcctcctcctcttcttcagccacctgatcctcagcgcctgggctcacg gggagaagcgtctcgcccccaaagggcaacccggacccgctgccactgataggaacc ctagaggctccagcagcagacagagcagcagtagcgctatgtcttcctcttctgcctcctc ctcccccgcagcttctctgggcagccaaggaagtggcttggagcagagcagtttccagtg gagcccctcggggcgccggaccggcagcctctactgcagagtgggcatcggtttccatct gcagatctacccggatggcaaagtcaatggatcccacgaagccaatatgttaagccaag ttcacagatga | 2 |
| FGF5-v1 | MSLSFLLLLFFSHLILSAWAHGEKRLAPKGQPGPAATDRNPRGS SSRQSSSSAMSSSSASSSPAASLGSQGSGLEQSSFQWSPSGR RTGSLYCRVGIGFHLQIYPDGKVNGSHEANMLSVLEIFAVSQGI VGIRGVFSNKFLAMSKKGKLHASAKFTDDCKFRERFQENSYNT YASAIHRTEKTGREWYVALNKRGKAKRGCSPRVKPQHISTHFL PRFKQSEQPELSFTVTVPEKKKPPSPIKPKIPLSAPRKNTNSVKY RLKFRFG | 3 |
| FGF5-v1_only CDS | atgagcttgtccttcctcctcctcctcttcttcagccacctgatcctcagcgcctgggctcacg gggagaagcgtctcgcccccaaagggcaacccggacccgctgccactgataggaacc ctagaggctccagcagcagacagagcagcagtagcgctatgtcttcctcttctgcctcctc ctcccccgcagcttctctgggcagccaaggaagtggcttggagcagagcagtttccagtg gagcccctcggggcgccggaccggcagcctctactgcagagtgggcatcggtttccatct gcagatctacccggatggcaaagtcaatggatcccacgaagccaatatgttaagtgttttg gaaatatttgctgtgtctcaggggattgtaggaatacgaggagttttcagcaacaaattttta gcgatgtcaaaaaaaggaaaactccatgcaagtgccaagttcacagatgactgcaagtt cagggagcgttttcaagaaaatagctataatacctatgcctcagcaatacatagaactga aaaaacagggcgggagtggtatgtggccctgaataaaagaggaaaagccaaacgag ggtgcagcccccgggttaaaccccagcatatctctacccattttctgccaagattcaagca gtcggagcagccagaactttctttcacggttactgttcctgaaaagaaaaagccacctagc cctatcaagccaaagattcccctttctgcacctcggaaaaataccaactcagtgaaataca gactcaagtttcgctttggataa | 4 |
| FGF5-full | gctcgggtggcctctctcttcccctctccccttctcttccccgaggctatgtccacccggtgcg | 5 |
| | gcgaggcgggcagagccagaggcacgcagccgcacaggggctacagagcccaga atcagccctacaagatgcacttaggacccccgcggctggaagaatgagcttgtccttcctc ctcctcctcttcttcagccacctgatcctcagcgcctgggctcacggggagaagcgtctcgc ccccaaagggcaacccggacccgctgccactgataggaaccctagaggctccagcag cagacagagcagcagtagcgctatgtcttcctcttctgcctcctcctcccccgcagcttctct gggcagccaaggaagtggcttggagcagagcagtttccagtggagcccctcggggcgc cggaccggcagcctctactgcagagtgggcatcggtttccatctgcagatctacccggatg gcaaagtcaatggatcccacgaagccaatatgttaagtgttttggaaatatttgctgtgtctc aggggattgtaggaatacgaggagttttcagcaacaaatttttagcgatgtcaaaaaaag gaaaactccatgcaagtgccaagttcacagatgactgcaagttcagggagcgttttcaag aaaatagctataatacctatgcctcagcaatacatagaactgaaaaaacagggcggga gtggtatgtggccctgaataaaagaggaaaagccaaacgagggtgcagcccccgggtt aaaccccagcatatctctacccattttctgccaagattcaagcagtcggagcagccagaa ctttctttcacggttactgttcctgaaaagaaaaagccacctagccctatcaagccaaagat tcccctttctgcacctcggaaaaataccaactcagtgaaatacagactcaagtttcgctttgg ataatattcctcttggccttgtgagaaaccattctttcccctcaggagtttctataggtgtcttca gagttctgaagaaaaattactggacacagcttcagctatacttacactgtattgaagtcacg tcatttgtttcaatgtgactgaaacaaaatgttttttgataggaaggaaactggaattctttgta ctaatacagggagcacactccttcagttcagcaagacataaagccttttgctttatgcttgag ggatatttagaactttgtattttcggaaagttaaataacagggactacgtatttttctgacttttac agattaacctgaaagaacatacatgatacatttttatttttggtttccaaagaatattttgatgca gataaaatattttgttaacttttgtttttttttgtttgttttcttaaaagtacctctgcattgagcatatttt cttacttttattattttaattaatatgacataagcaatcattttatgctgtttatgaattataaatgtgt ttatagctcatttgtaatatggaaatcttttacatttttcctattcactgcacttttttattgtttttatttct agccatacctcagataatatgtttagttttacattttaaaatgtttaaattctctttcacagcacca aaggctcagcttggatttgtgtgtatgtgtatgtcaattcatgacattatgtggaatcctaaacc tttggtggctgggatatgatgggttagaagcaaggagaaaatataaggactttttgatgga attaaatgtgggaggtaaggaaaaggatttagaggtaaaagtacactaagtttgcaacatt tattgagatctaagtctgtcttgccttcatttctctttttatctcccccttgccctcattcttgaacag ctggaggaatacattttattctgtccatgaagcatacactatgaaattcaagtgcttaaaaat acttctatgactctctgctatcccactgtatagatccacagggagcaaacacttagaaatga tagagaactgaaggagatcaatggtttaacagttatccatgccaagtcccattgtcagaaa tattcttattactcagtcaaacactctttgagcttcccttcctaaaggtaaccaatccagtgaat agatgtgcccttttataaggaaacttctgatgtttattaaaaaaactggccttttgatagaggta acttaatttgggaatttgttgtgttgaaatggcatttaatttcaacctaaatactgactgctggac ataaatcacagaaaatttaacttaagaaaatttacaaaatttattctcaggtaatcattttaat aaagttctgcaaaatacacgtttatcttacattcagaaatgtggcaaaaaaggcatagcta aaggctaaacatatggctttagtagtaacaaaagggttcatagaaacttcatggtttgcattt aaacatgtttaaagtgtacttataaactatttttttcttaaagcaaactatgatttattttggtgcac aaatacaaagtggaaacttaccaaaattgaactagctaccatataagcagattgctttaatt tgatgggaaaatagtacacacatatatataacaaataatatattaaaaaacccatccatca actaaaacattatatgtatacatcagtatagtgttttattataaagccaattatctgattaagcat tctttccactgaatgcataatgtttaaatagcataaaatgaaatgctacaaaaattgaactaa tttatactttaaagtatttctgggttaaatgaaacaatgaaattttttagtatgttcaactctcatcc aaatggcatatgaccctgtttacacagcctaaagctaaaaatattactctagtttattctaatct | |
| | attgttaagtattgtgcactgtataccaagttcttagggcacatgaaaaattttagctgccaaa caggaactagtaaacatatgttcctaataagtgaagggaaagataataatgatggtcaac aataagccacgtcaatgcataagttgtataggctaaatgttgcttgtaggctacattaaactc aaatgtaatagtttatcttatactcctggtttgatttgattagcatattaacgtgaaagtaggata gctactaaatatatattatgcaagtcaggaatcattaatttcaaaatttaaagccatgctaaa attaaaaagaaaatattaaattacacaattacacttgtctttactggccatacaaaatgattttt tttttttttttgagacagagtcttgctctgtcaccaggctggagtgcagtggcatgatctcggctc actgcaacctccaactccctggtttaagggattctcctgcctcagcctcccaagtagctggg attacagactcatgccaccacgccagctaatttttgtatttttagtagagacggggtttcacca tgttggtcaggatggtctcaatcctggcctcttgatagtcctgacctcatgatctgcccacctc ggcctccccaaagtgctgggattacaggtacaatgatgtataattaatgcttagtgaagcat aaagttacctacatcaattaattaaatgaacttatgtacagaaaacatgtataaatataagt ctatactaatgcttacaactttctaagagggttcttgcttatgtagctttttattattttaagtaacta gaaccaccaaatatcaaataaaattatttggttatggttatgttcatctaaacacaacaataa cttttatattaatatttaggagtctattttgtctataggtgacaaacatctccagactaacatgtc agttttatcaattatattatgtttaattatttaagatttctttatgtggaacatctatagagataaata gaaattttcaataagatgtagtaacactgtgatttatctttcaagagtctctcttcacttccttcta aagagactaatttgagagtacaggtgcatattaattttcttggttctttcagctgaattatattgg tccagaagttcaaaatcatgtgacaataataagggatactgacagaagttatttccaagttt gtgtatatattataaaaattacatatataaaactaaggcttttatttctgttatttttaagcttttattt cttgtagctaaaaataaaacatcataaatctggtaggtaaatttcttattaaatcaatcttgaa atagaaaatgtaataactttcttaccattaacattttttacccttccatagaagggagggaata aatcatgacttatcccattttcaataacaaaacgaaactatggcactaaccaaaaacttgc attctggcataatttttacagttgcagagaattgtttctgggctcattaaaaaaagtagtattgc agacattgctgcaatgggaagcagacaataacttcttaaaggaattctacacctcctttaag atttacttaattgctacatctaaattctgataatttaaaatccattttaggtgataaaattttttaaa agttttgaaggaaacctctggataaatggacaaggcctaatttttttttgtagtcaatccaact gtactggccaatttttgaaataagattatatgattaggtattagcagagacaaagagttacct cctccatcttactctgccctatttgaaagtctcaggggagaaaagggaacaagatgctgat ccaacctgagtggagtcaggtgaggcatctttacatctaagaatttttttttaaattttattattatt atacttcaagttctagggtacatgtccacaatgcacatgtctgtcacacatgcacacatgtg ccatgctggtgtgctgcacccaccaacctgtcatccagcattaggtatatctcctaatgctat ccctcccctctccacccaccccacagcaggccccggtatgtgatgttccccttcgtgtgtcc atgtgttcttattgttcaattcccacctatgagtgagaatatgtggtgtttggtttttggtccttgca atagtttgctgagaatgatggtttccagcttcatccatgtccctacaaagaacatgaactcat cattttttatggctgcatagtattccatggtgtatatgtgccacattttcttaatccagtctatcattg ttggacatttgggttggttccaagtctttgctattgtgaatagtgctgcaataaacatatgtgtgc atgtgtcttta | |
| FGF5-v3 | MSKKGKLHASAKFTDDCKFRERFQENSYNTYASAIHRTEKTGR EWYVALNKRGKAKRGCSPRVKPQHISTHFLPRFKQSEQPELSF TVTVPEKKKPPSPIKPKIPLSAPRKNTNSVKYRLKFRFG | 6 |
| FGF5-v3_only CDS | atgtcaaaaaaaggaaaactccatgcaagtgccaagttcacagatgactgcaagttcag ggagcgttttcaagaaaatagctataatacctatgcctcagcaatacatagaactgaaaa aacagggcgggagtggtatgtggccctgaataaaagaggaaaagccaaacgagggt gcagcccccgggttaaaccccagcatatctctacccattttctgccaagattcaagcagtc | 7 |
| | ggagcagccagaactttctttcacggttactgttcctgaaaagaaaaagccacctagccct atcaagccaaagattcccctttctgcacctcggaaaaataccaactcagtgaaatacaga ctcaagtttcgctttggataa | |
| FGF5-full | MSLSFLLLLFFSHLILSAWAHGEKRLAPKGQPGPAATDRNPRGS SSRQSSSSAMSSSSASSSPAASLGSQGSGLEQSSFQWSPSGR RTGSLYCRVGIGFHLQIYPDGKVNGSHEANMLSVLEIFAVSQGI VGIRGVFSNKFLAMSKKGKLHASAKFTDDCKFRERFQENSYNT YASAIHRTEKTGREWYVALNKRGKAKRGCSPRVKPQHISTHFL PRFKQSEQPELSFTVTVPEKKKPPSPIKPKIPLSAPRKNTNSVKY RLKFRFG | 8 |
| α-SMA FW primer | TGC TGA CAG AGG CAC CAC TGA A | 9 |
| α-SMA RV primer | CAG TTG TAC GTC CAG AGG CAT AG | 10 |
| mCol1α1 FW primer | CCT CAG GGT ATT GCT GGA CAA C | 11 |
| mCol1α1 RV primer | CAG AAG GAC CTT GTT TGC CAG G | 12 |
| Fibronectin FW primer | CCC TAT CTC TGA TAC CGT TGT CC | 13 |
| Fibronectin RV primer | TGC CGC AAC TAC TGT GAT TCG G | 14 |
| Vimentin FW primer | CGG AAA GTG GAA TCC TTG CAG G | 15 |
| Vimentin RV primer | AGC AGT GAG GTC AGG CTT GGA A | 16 |
| mHPRT FW primer | CTG GTG AAA AGG ACC TCT CGA AG | 17 |
| mHPRT RV primer | CCA GTT TCA CTA ATG ACA CAA ACG | 18 |
| Primer-1 | AAAGGTACCATGAGCTTGTCCTTCCTCCTC | 19 |
| Primer-2 | AAAAAGCTTTCACTTATCGTCGTCATCCTTGTAATCTCTGTGA ACTTGGCTTAACATATTGGCTTCGT | 20 |

### [Industrial Applicability]

FGF5-v2, which is a transcript variant of fibroblast growth factor 5 (FGF5), and a protein thereof according to the present disclosure were developed by adjusting the amino acid sequence of full-length FGF5. It was confirmed that the transcript variant and the protein thereof have effects of inhibiting the progression of liver fibrosis and improving liver function abnormalities accompanying liver fibrosis, etc. and various increased inflammation- and fibrosis-related cytokines, etc. Therefore, it is expected to achieve the effects of preventing, improving, and treating various diseases comprising cirrhosis or liver cancer, etc. by suppressing liver fibrosis by administering, to a patient, a gene encoding FGF5-v2, the transcript thereof, the protein thereof, and/or various carriers (e.g., AAV) loading the same, and thus has industrial applicability.

## Claims

1. A FGF5-v2 protein consisting of an amino acid sequence of SEQ ID NO: 1.

2. A nucleic acid molecule encoding the FGF5-v2 protein of claim 1.

3. The nucleic acid molecule of claim 2, wherein the FGF5-v2 gene comprises a nucleotide sequence of SEQ ID NO: 2.

4. An expression vector comprising the nucleic acid molecule of claim 2 or 3.

5. An isolated cell into which the expression vector of claim 4 is introduced.

6. A pharmaceutical composition for preventing or treating liver fibrosis, the pharmaceutical composition comprising, as an active ingredient, an FGF5-v2 protein consisting of an amino acid sequence of SEQ ID NO: 1; a nucleic acid molecule encoding the FGF5-v2 protein; an expression vector comprising the nucleic acid molecule; or an isolated cell, into which the expression vector is introduced.

7. The pharmaceutical composition of claim 6, wherein the FGF5-v2 protein, the nucleic acid molecule, the expression vector, or the cell is loaded on a carrier.

8. The pharmaceutical composition of claim 7, wherein the composition comprises the FGF5-v2 protein, the nucleic acid molecule, or the expression vector as an active ingredient, and the carrier is one or more selected from the group consisting of a viral particle, a vesicle, a nanoparticle, a microparticle, a liposome, a transposon, a micelle, an antibody, and an exosome.

9. The pharmaceutical composition of claim 6, wherein the liver fibrosis is liver fibrosis in one or more selected from the group consisting of alcoholic fatty liver disease, non-alcoholic fatty liver disease, hepatitis C, hepatitis B, autoimmune hepatitis, hepatic encephalopathy, primary biliary cirrhosis, liver cancer, and hepatocellular carcinoma.

10. The pharmaceutical composition of claim 9, wherein the non-alcoholic fatty liver disease is any one or more selected from the group consisting of non-alcoholic steatohepatitis (NASH), non-alcoholic fatty liver (NAFL), hepatic steatosis, acute fatty liver of pregnancy (AFLP), NAFLD-related liver failure, NAFLD-related liver fibrosis, NAFLD-related hepatocirrhosis, and NAFLD-related liver cancer.

11. The pharmaceutical composition of claim 6, wherein the composition is administered in combination with an anti-inflammatory agent.

12. The pharmaceutical composition of claim 11, wherein the anti-inflammatory agent is a steroidal anti-inflammatory agent or a non-steroidal anti-inflammatory agent.

13. A method of preventing or treating liver fibrosis, the method comprising the step of administering an FGF5-v2 protein consisting of an amino acid sequence of SEQ ID NO: 1; a nucleic acid molecule encoding the FGF5-v2 protein; an expression vector comprising the nucleic acid molecule; or an isolated cell, into which the expression vector is introduced, to an individual in need thereof.

14. Use of a composition in preventing or treating liver fibrosis, the composition comprising, as an active ingredient, an FGF5-v2 protein consisting of an amino acid sequence of SEQ ID NO: 1; a nucleic acid molecule encoding the FGF5-v2 protein; an expression vector comprising the nucleic acid molecule; or an isolated cell, into which the expression vector is introduced.

15. Use of a composition in preparing a prophylactic or therapeutic agent for liver fibrosis, the composition comprising, as an active ingredient, an FGF5-v2 protein consisting of an amino acid sequence of SEQ ID NO: 1; a nucleic acid molecule encoding the FGF5-v2 protein; an expression vector comprising the nucleic acid molecule; or an isolated cell, into which the expression vector is introduced.
